# EUROPEAN PATENT SPECIFICATION

(11) **EP 3 532 493 B1**
(45) Date of publication and mention of the grant of the patent: **26.04.2023**
(21) Application number: 17804018.4
(22) Date of filing: 27.10.2017
(51) Int. Cl.: C07K 14/81, C12N 5/071, C12N 15/113, C12N 15/86

(54) **GENETICALLY MODIFIED MOUSE MODEL FOR HUMAN HEPATOCYTE XENOTRANSPLANTATION**
GENETISCH MODIFIZIERTES MAUSMODELL FÜR XENOTRANSPLANTATION HUMANER HEPATOZYTEN
SOURIS GÉNÉTIQUEMENT MODIFIÉE POUR XÉNOTRANSPLANTATION D'HÉPATOCYTE HUMAIN

(30) Priority: 27.10.2016 US 201662413736 P; 27.10.2016 US 201662413743 P
(43) Date of publication of application: 04.09.2019
(73) Proprietor: The Jackson Laboratory, Bar Harbor, ME 04609 (US)
(72) Inventor: SHULTZ, Leonard, D., Bar Harbor ME 04609 (US)
(74) Representative: Potter Clarkson
(86) International application number: PCT/US2017/058758
(87) International publication number: WO 2018/081561

(56) References cited:
- WO-A2-2012/145624
- Johnny Huard: "New Advanded Technology in Stem Cell Therapy", , November 2014 (2014-11), XP055435052, Retrieved from the Internet: URL:http://www.dtic.mil/get-tr-doc/pdf?AD= ADA613649 [retrieved on 2017-12-14]
- Anonymous: "Humanized Liver Mouse Model - Mueller Lab for Gene Therapy | Horae Gene Therapy Center | University of Massachusetts Medical School", , 21 September 2016 (2016-09-21), XP055435060, Retrieved from the Internet: URL:https://web.archive.org/web/2016092111 0122/https://www.umassmed.edu/muellerlab/t echnology/humanized-liver-mouse/ [retrieved on 2017-12-14]
- SHAOYONG LI ET AL: "Efficient and Targeted Transduction of Nonhuman Primate Liver With Systemically Delivered Optimized AAV3B Vectors", MOLECULAR THERAPY, vol. 23, no. 12, December 2015 (2015-12), pages 1867-1876, XP055435148, US ISSN: 1525-0016, DOI: 10.1038/mt.2015.174
- SONG XIJUN ET AL: "A Mouse Model of Inducible Liver Injury Caused by Tet-On Regulated Urokinase for Studies of Hepatocyte Transplantation", AMERICAN JOURNAL OF PATHOL; [10640], ELSEVIER INC, US, vol. 175, no. 5, November 2009 (2009-11), pages 1975-1983, XP009144942, ISSN: 0002-9440, DOI: 10.2353/AJPATH.2009.090349
- STOCK PEGGY ET AL: "The generation of hepatocytes from mesenchymal stem cells and engraftment into murine liver", NATURE PROTOCOLS, NATURE PUBLISHING GROUP, GB, vol. 5, no. 4, April 2010 (2010-04), pages 617-627, XP009177563, ISSN: 1750-2799, DOI: 10.1038/NPROT.2010.7
- MIGNON A ET AL: "SELECTIVE REPOPULATION OF NORMAL MOUSE LIVER BY FAS/CD95-RESISTANT HEPATOCYTES", NATURE MEDICINE, NATURE PUB. CO, vol. 4, no. 10, October 1998 (1998-10), pages 1185-1188, XP000907504, ISSN: 1078-8956, DOI: 10.1038/2681
- JIANQIANG DING ET AL: "Spontaneous hepatic repopulation in transgenic mice expressing mutant human [alpha]1-antitrypsin by wild-type donor hepatocytes", JOURNAL OF CLINICAL INVESTIGATION, vol. 121, no. 5, 2 May 2011 (2011-05-02), pages 1930-1934, XP055435057, US ISSN: 0021-9738, DOI: 10.1172/JCI45260

## Description

### TECHNICAL FIELD

The present disclosure generally relates to mouse models. More specifically, the present disclosure relates to transgenic mouse models receptive to human liver cell xenotransplantation.

### BACKGROUND

Animal models are often used in laboratory research to simulate, mimic, or inform researchers of human biological activity and disease. There is a continuing need for mice models of human liver function and disease.

Huard, 2011 is entitled "New Advanced Technologies In Stem Cell Therapy". A publication by the University of Massachusetts Medical School, published on 21 September 2016 is entitled "Humanized Liver Mouse Model - Mueller Lab for Gene Therapy | Horae Gene Therapy Center | University of Massachusetts Medical School". Li et al., 2015 is entitled "Efficient and Targeted Transduction of Nonhuman Primate Liver With Systemically Delivered Optimized AAV3B Vectors". Song et al., 2009 is entitled "A Mouse Model of Inducible Liver Injury Caused by Tet-On Regulated Urokinase for Studies of Hepatocyte Transplantation". Stock et al., 2010 is entitled "The generation of hepatocytes from mesenchymal stem cells and engraftment into murine liver". Mignon et al., 1998 is entitled "Selective Repopulation of Normal Mouse Liver by Fas/Cd95-Resistant Hepatocytes". WO 2012/145624 A2 is entitled "rAAV-Based Compositions and Methods for Treating Alpha-1 Anti-Trypsin Deficiencies".

### SUMMARY

The invention relates to a method comprising: administering to an immunodeficient genetically-modified mouse a molecular anti-hepatocyte agent, wherein the molecular anti-hepatocyte agent is monocrotaline or anti-mouse CD95 antibody, wherein the immunodeficient mouse expresses a PiZ variant (Glu342Lys) of human α-1 antitrypsin (Z-AAT), and administering human hepatocytes to the mouse. Optionally, the human hepatocytes are primary hepatocytes. Administering human hepatocytes to a genetically-modified immunodeficient mouse of the present disclosure optionally includes injecting the hepatocytes. Administering human hepatocytes to a genetically-modified immunodeficient mouse of the present disclosure optionally includes intrasplenic administration of the hepatocytes. The administered hepatocytes optionally express a marker protein such as green fluorescent protein or a fluorescent analogue thereof.

The genetically-modified immunodeficient mouse may be an immunodeficient NSG, NRG or NOG mouse.

The administered hepatotoxin is monocrotaline according to particular embodiments. According to aspects of the present disclosure, a method of making a genetically-modified immunodeficient mouse comprising engrafted exogenous hepatocytes includes administering about 10-100 mg/kg monocrotaline to the genetically-modified immunodeficient mouse prior to administering human hepatocytes.

According to aspects of the present disclosure, treating the genetically-modified immunodeficient mouse to reduce endogenous mouse hepatocytes is performed prior to administering the human hepatocytes and includes administering an anti-mouse CD95 antibody to the genetically-modified immunodeficient mouse. According to particular aspects of the present disclosure, treating the genetically-modified immunodeficient mouse to reduce endogenous mouse hepatocytes is performed prior to administering the human hepatocytes and includes administering about 0.1 - 10 µg of the anti-mouse CD95 antibody to the genetically-modified immunodeficient mouse.

### BRIEF DESCRIPTION OF THE DRAWINGS

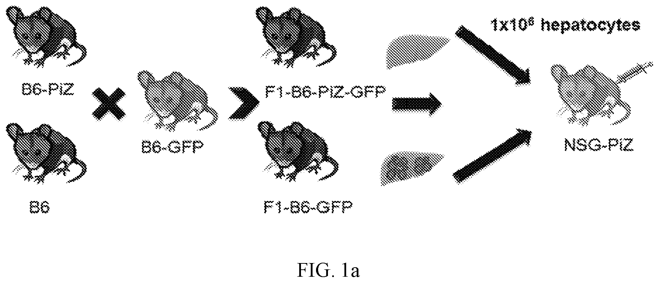

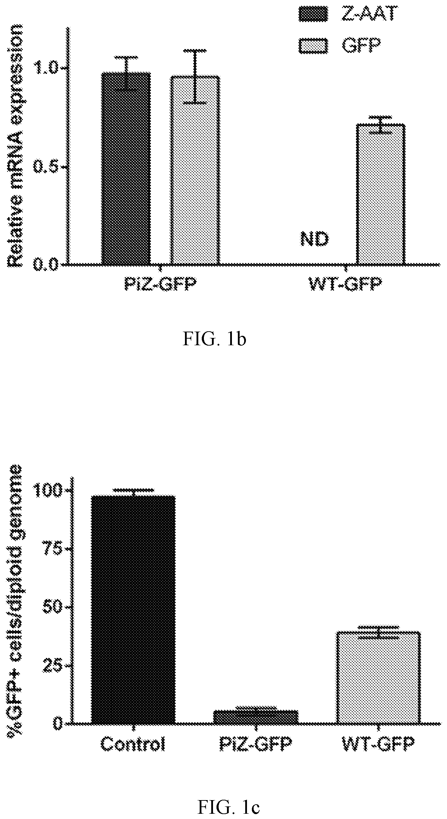

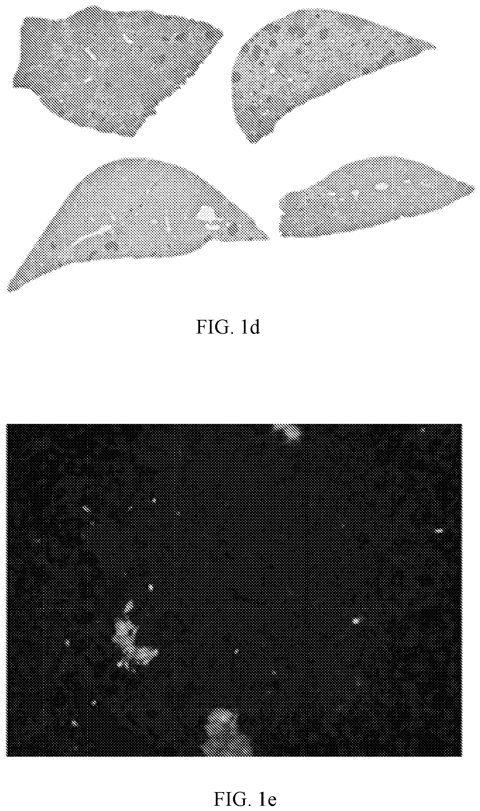

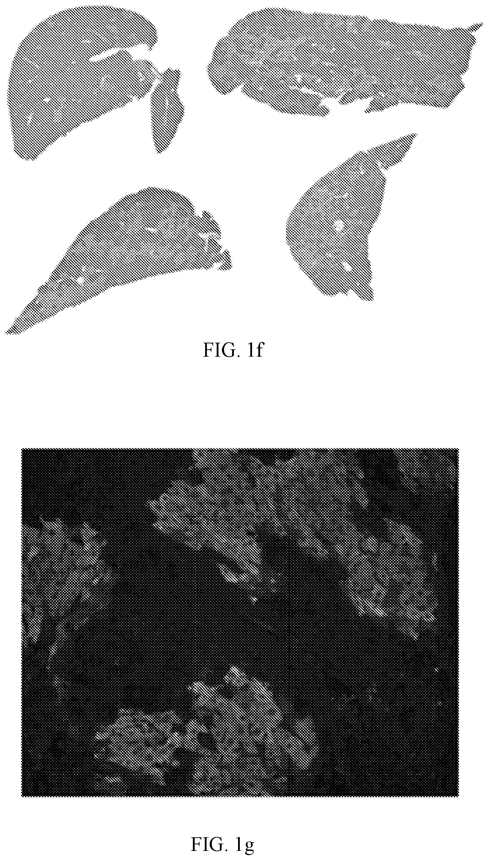

Figure 1 consists of seven panels labeled panel a, b, c, d, e, f, and g. Panel **(a)** of Figure 1 (Figure 1a) shows the development of two donor mice strains: B6-PiZ-GFP, which has GFP-labeled mouse hepatocytes expressing PiZ, and B6-GFP, which has GFP-labeled wild-type mouse hepatocytes. The GFP-expressing hepatocytes from donor mice were isolated and engrafted into immunocompromised male NSG-PiZ mice, which express the human PiZ gene, by the intrasplenic injection of 1,000,000 cells per mouse. The recipient mice were euthanized 8 weeks post-engraftment, and liver tissue was obtained for subsequent analysis. Panel **(b)** of Figure 1 (Figure 1b) is a graph depicting the relative mRNA of Z-AAT (dark gray) and GFP (light gray) observed in PiZ-GFP donor cells and wild-type, GFP donor cells (WT-GFP) as determined by RT-qPCR. Error bars correspond to standard error of the mean - 3 mice per group. Panel **(c)** of Figure 1 (Figure 1c) is a graph depicting the percent GFP-positive cells per diploid genome in NSG-PiZ mice engrafted with PiZ-GFP hepatocytes (n=3) or WT-GFP hepatocytes (n=4) as determined by ddPCR performed on genomic DNA. Genomic DNA from WT-GFP donor liver was used as a positive control (n=2). Error bars correspond to standard error. Panel **(d)** of Figure 1 (Figure 1d) is an image of an anti-GFP immunostained liver section from a NSG-PiZ mouse that received a PiZ-GFP hepatocyte graft. Panel **(e)** of Figure 1 (Figure 1e) is a fluorescence image of a NSG-PiZ mouse liver section that received a PiZ-GFP hepatocyte graft. Panel **(f)** of Figure 1 (Figure 1f) is an image of an anti-GFP immunostained liver section from a NSG-PiZ mouse that received a WT-GFP hepatocyte graft, which displays increased staining relative to Figure 1d. Panel **(g)** of Figure 1 (Figure 1g) is a fluorescence image of a NSG-PiZ mouse liver section that received a WT-GFP hepatocyte graft, which displays increased fluorescence relative to Figure 1e.

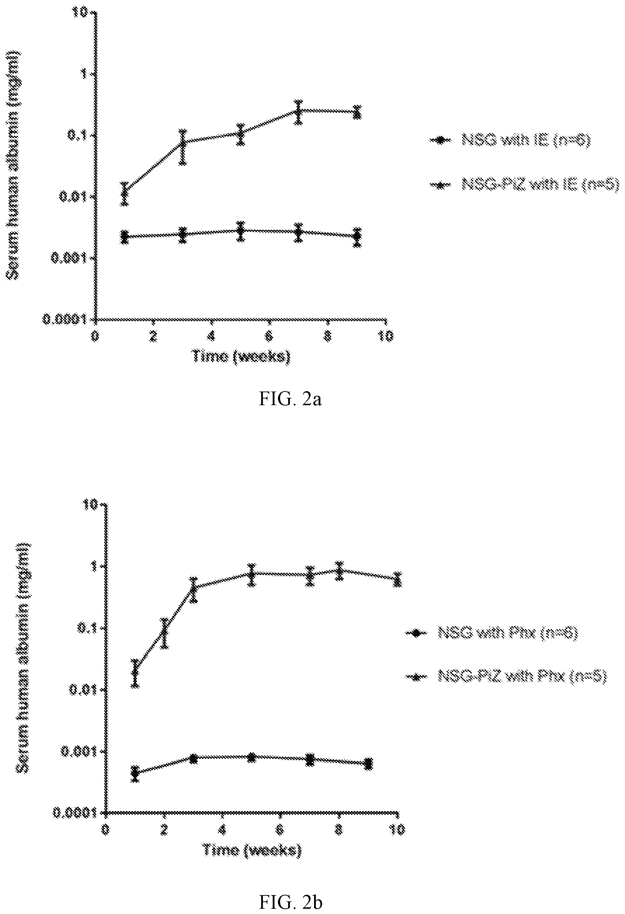

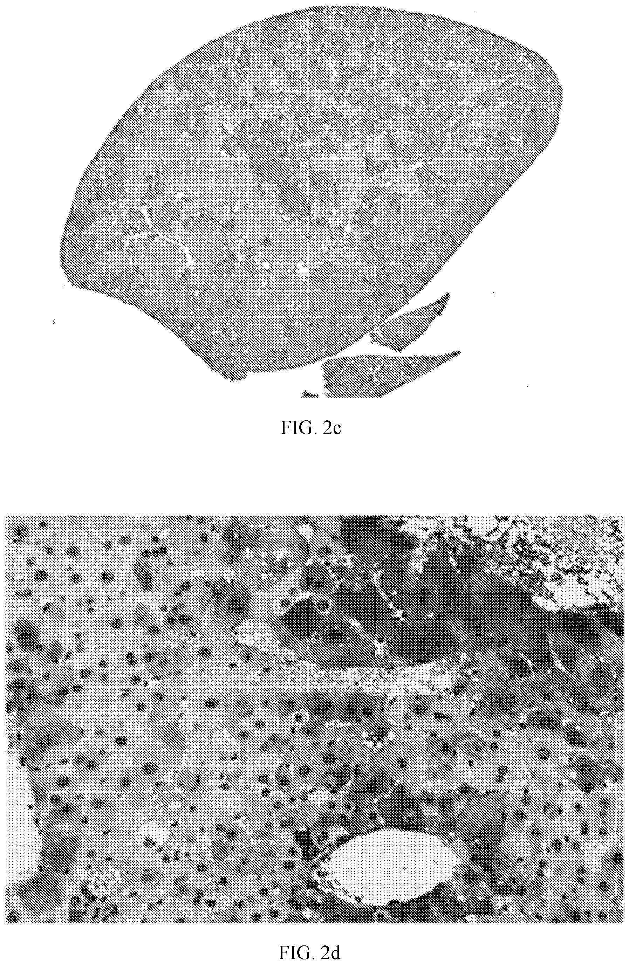

Figure 2 consists of four panels labeled panel a, b, c, and d. Panel (**a**) of Figure 2 (Figure 2a) is a graph depicting the average concentration of serum human albumin measured in either NSG mice (circles; 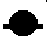 ) or NSG-PiZ mice (triangles; 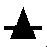 ) that underwent intrasplenic engraftment with 1,000,000 mature human hepatocytes. The x-axis corresponds to time in weeks, and the y-axis corresponds to serum human albumin concentration in mg/mL on a logarithmic scale. Error bars correspond to standard error. The NSG-PiZ mice displayed significantly more human serum albumin than the NSG mice at all timepoints. Panel (**b**) of Figure 2 (Figure 2b) is a graph depicting the concentration of serum human albumin measured in either NSG mice (circles; 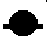 ) or NSG-PiZ mice (triangles; 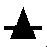 ) that underwent a partial hepatectomy (Phx) followed by intrasplenic engraftment with 1,000,000 mature human hepatocytes. The x-axis corresponds to time in weeks, and the y-axis corresponds to serum human albumin concentration in mg/mL on a logarithmic scale. Error bars correspond to standard error. The NSG-PiZ mice displayed significantly more human serum albumin than the NSG mice at all timepoints. Panels **(c)** (Figure 2c) and **(d)** (Figure 2d) of Figure 2 are images of NSG-PiZ mouse liver 10 weeks after partial hepatectomy and intrasplenic engraftment with 1,000,000 mature human hepatocytes shown with immunostaining against human albumin.

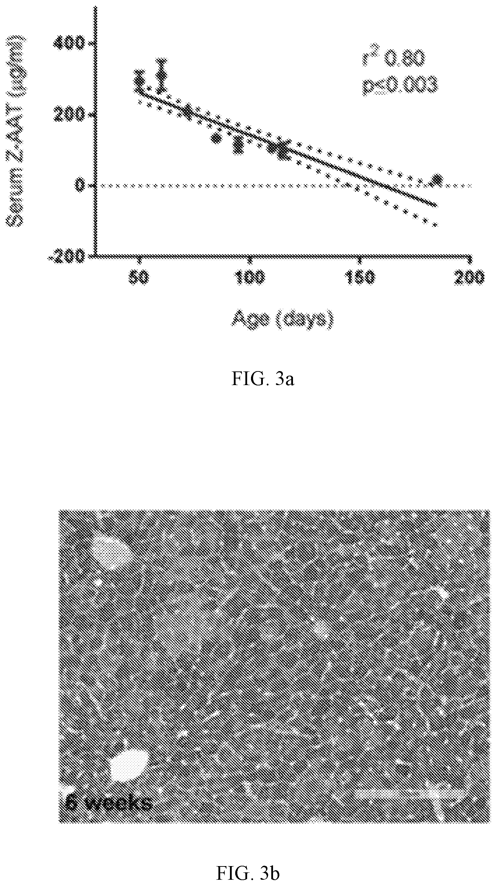

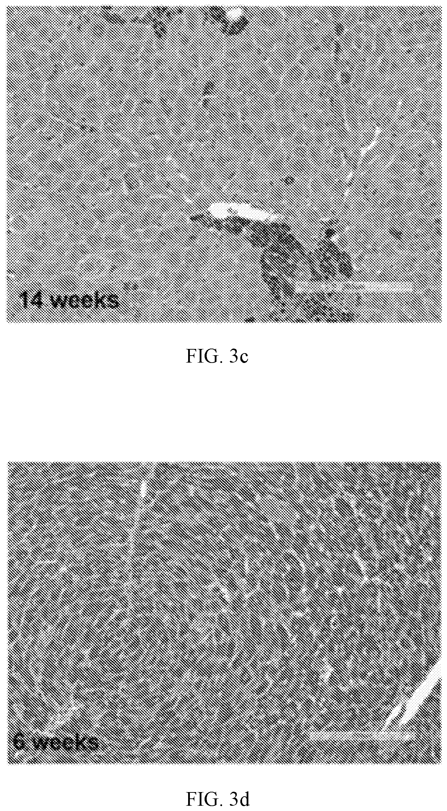

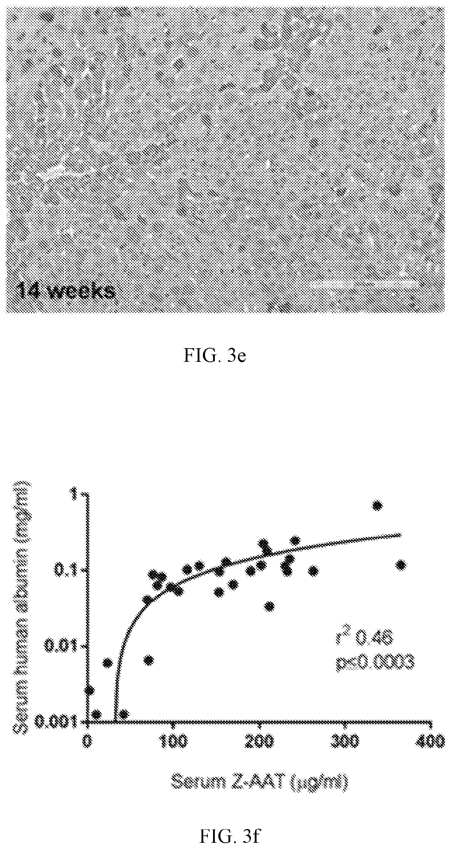

Figure 3 consists of six panels labeled panel a, b, c, d, e, and f. Panel (a) of Figure 3 (Figure 3a) is a graph depicting the serum Z-AAT concentration of NSG-PiZ mice as quantified by ELISA. The x-axis corresponds to the age of the mice in days, and the y-axis corresponds to the average serum Z-AAT concentration measured in the mice. Error bars correspond to standard error. The 50 day group consisted of 10 mice, 60 days n=9, 72 days n=27, 85 days n=12, 95 days n=17, 110 days n=7, 115 days n=3, and 185 n=5. Panel **(b)** of Figure 3 (Figure 3b) is a representative image of a NSG-PiZ mouse liver section stained using diastase resistant periodic acid-Schiff (PASD) at 6 weeks age. Increased staining correlates with increased globules (*e.g.*, globules having Z-AAT aggregate). Panel **(c)** of Figure 3 (Figure 3c) is a representative image of a NSG-PiZ mouse liver section stained using PASD at 14 weeks age, which displays less staining relative to 6 weeks. Panel **(d)** of Figure 3 (Figure 3d) is a representative image of a NSG-PiZ mouse liver section immunostained with an anti-human AAT antibody at 6 weeks age. Panel **(e)** of Figure 3 (Figure 3e) is a representative image of a NSG-PiZ mouse liver section immunostained with an anti-human AAT antibody at 14 weeks age, which displays less staining relative to 6 weeks suggesting less Z-AAT aggregation. Panel **(f)** of Figure 3 (Figure 3f) is a graph depicting a correlation between the measured serum human albumin concentration and the measured serum human Z-AAT concentration as quantified by ELISA in NSG-PiZ mice at the time of engraftment with human hepatocytes and 7-weeks post-engraftment. Each datapoint corresponds to one of thirty mice.

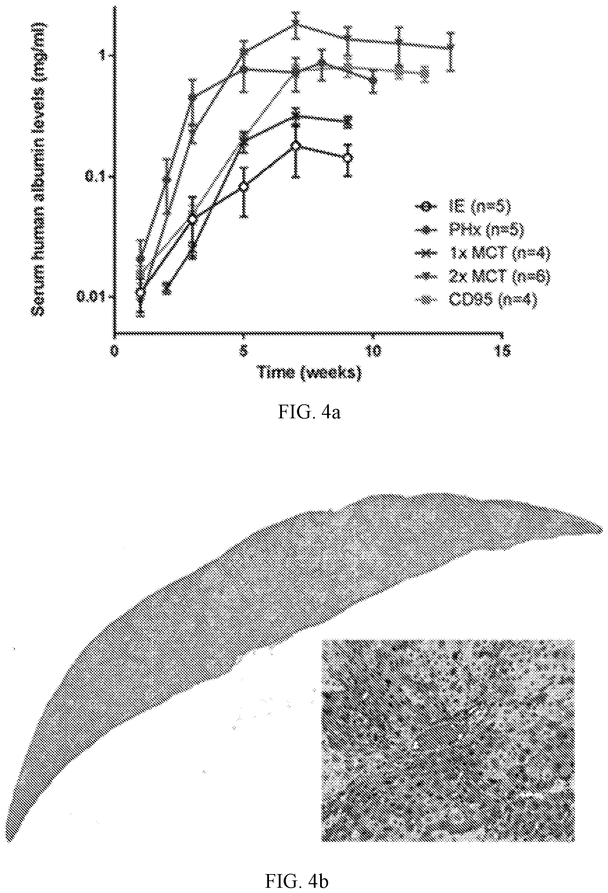

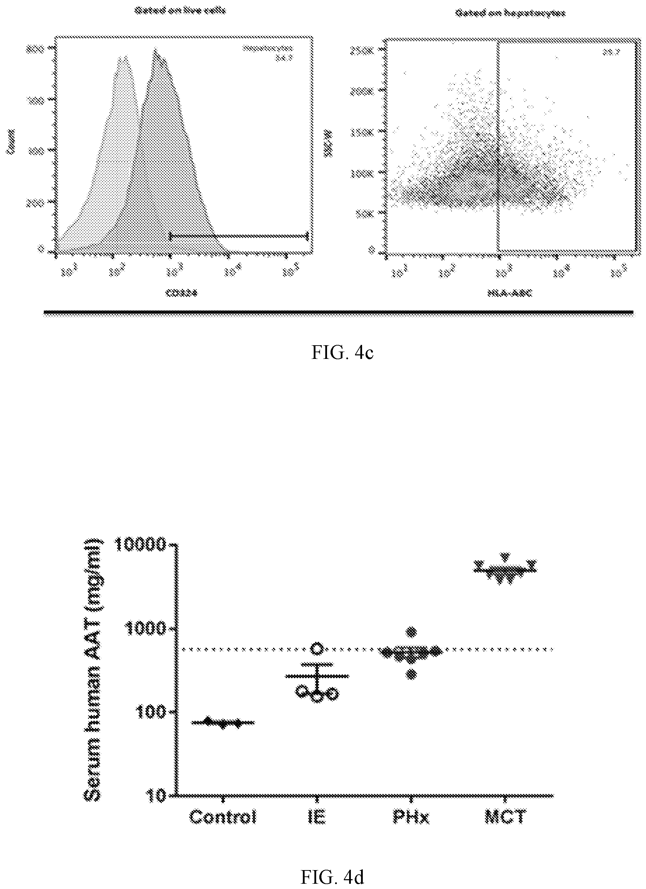

Figure 4 consists of four panels labeled panel a, b, c, and d. Panel **(a)** of Figure 4 (Figure 4a) is a graph depicting the average serum human albumin concentration of groups of NSG-PiZ mice that were each administered human hepatocytes. The x-axis corresponds to time in weeks and the y-axis corresponds to serum human albumin concentration in mg/mL on a logarithmic scale. Open circles ( 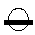 ) correspond to the average serum human albumin concentration measured in five NSG-PiZ mice that received intrasplenic engraftment (IE) without providing an additional selective disadvantage for the human hepatocytes. Closed circles ( 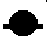 ) correspond to the average serum human albumin concentration measured in five NSG-PiZ mice that received intrasplenic engraftment following partial hepatectomy (PHx). X's ( 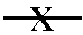 ) correspond to the average serum human albumin concentration measured in four NSG-PiZ mice that received intrasplenic engraftment seven days after receiving a single dose of 50 mg/kg intraperitoneal monocrotaline (1x MCT). Triangles ( 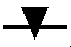 ) correspond to the average serum human albumin concentration measured in six NSG-PiZ mice that received intrasplenic engraftment after receiving a two doses of 50 mg/kg intraperitoneal monocrotaline fourteen and seven days before engraftment (2x MCT). Squares ( 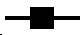 ) correspond to the average serum human albumin concentration measured in four NSG-PiZ mice that received intrasplenic engraftment along with 1 µg anti-mouse CD95 antibody at the time of engraftment (CD95). Error bars correspond to standard error. Mice receiving two doses of 50 mg/kg intraperitoneal monocrotaline displayed the greatest serum human albumin concentrations. Panel **(b)** of Figure 4 (Figure 4b) is an image of a mouse liver section obtained 10 weeks post-engraftment from a NSG-PiZ mouse that received 50 mg/kg intraperitoneal monocrotaline prior to engraftment. The section was stained using an anti-human albumin antibody. Panel **(c)** of Figure 4 (Figure 4c) consists of two flow cytometry plots performed on a mouse that received a human hepatocyte xenograft. The right flow cytometry plot is a scatter plot having an x-axis corresponding to human HLA-ABC⁺ fluorescence signal intensity and a y-axis corresponding to side scatter pulse width. 25.7% of the cells were gated as human HLA-ABC⁺ as shown. The CD324⁺ fluorescence signal intensity of the gated and ungated cells are shown in the right trace and left trace of the left flow cytometry plot, respectively. The left flow cytometry plot is a histogram having an x-axis corresponding to CD324⁺ fluorescence signal intensity and a y-axis corresponding to cell counts. Panel **(d)** of Figure 4 (Figure 4d) is a chart depicting the measured serum human AAT concentration in mg/mL (y-axis) in various mice at 10 weeks after engraftment with human hepatocytes. The x-axis labels correspond to different mouse groups, and each datapoint corresponds to a different mouse. Solid, horizontal lines depict mean values for each group, and error bars correspond to standard error. The dotted line was placed at 572 µg/mL. "Control" mice are NSG-PiZ mice that did not receive a hepatocyte xenograft. "IE" mice are NSG-PiZ mice that received intrasplenic engraftment (IE) without providing an additional selective disadvantage to mouse hepatocytes. "PHx" mice are NSG-PiZ mice that received intrasplenic engraftment following partial hepatectomy. "MCT" mice are NSG-PiZ mice that received intrasplenic engraftment after receiving 50 mg/kg intraperitoneal monocrotaline.

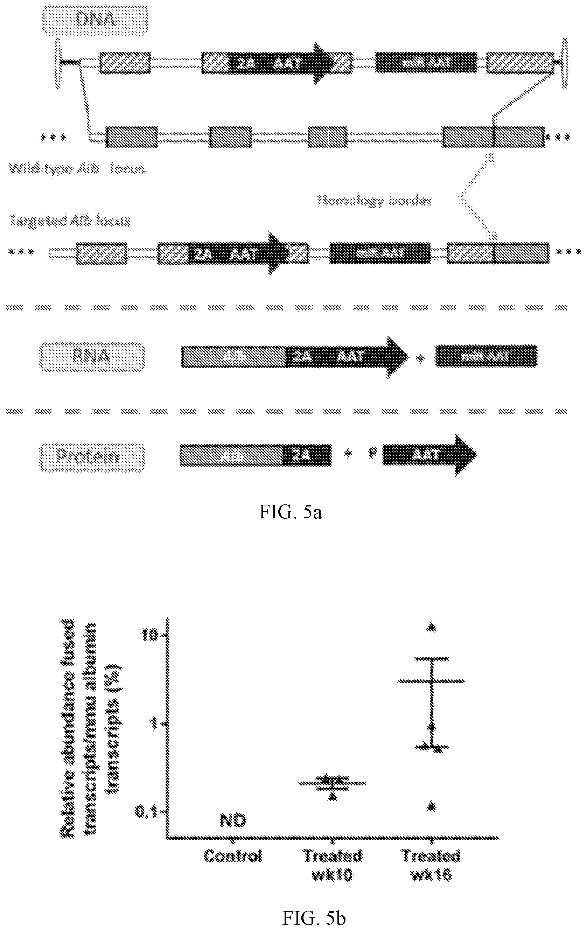

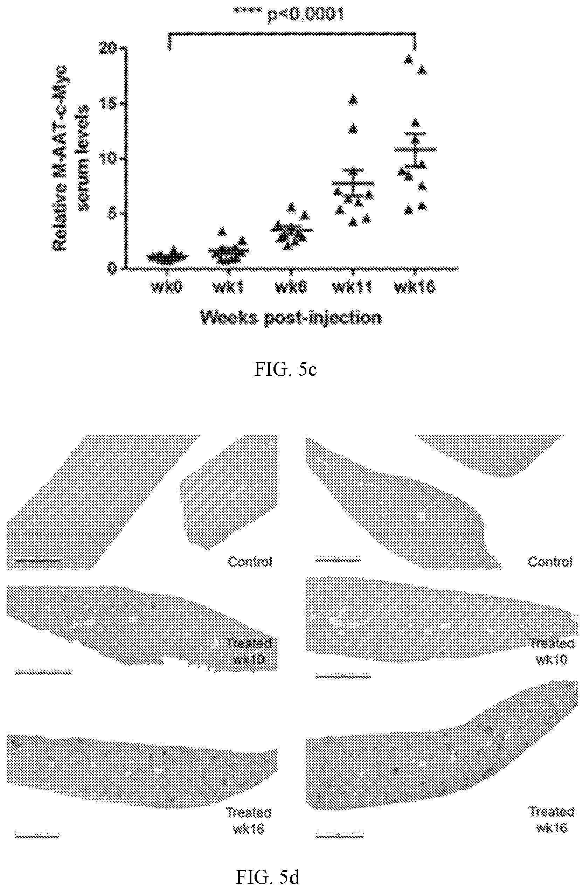

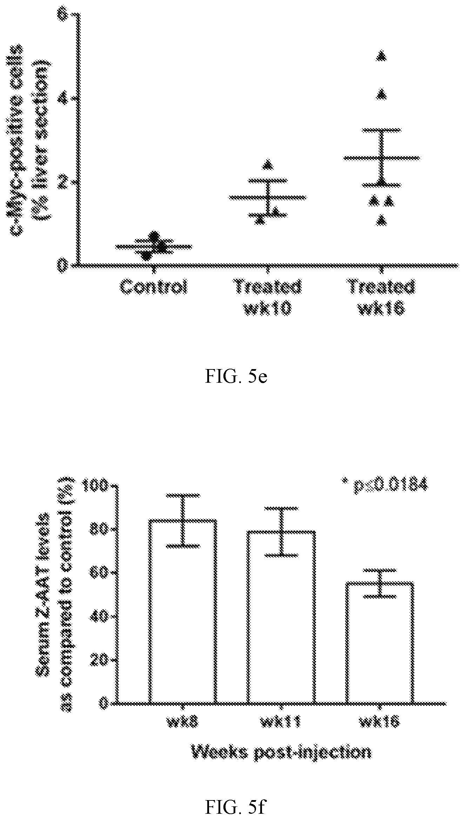

Figure 5 consists of six panels labeled panel a, b, c, d, e, and f. Panel **(a)** of Figure 5 (Figure 5a) is a cartoon depicting the organization of a recombinant AAV8 vector packaged with a promoterless, dual-function cassette containing an artificial miRNA that targets human AAT and a miRNA-detargeted human AAT sequence with a c-Myc tag. The cassette is flanked by 1.1-1.3 kilobase homology arms to the albumin locus. Panel **(b)** of Figure 5 (Figure 5b) is a graph depicting albumin-alpha-1 antitrypsin fused transcript / murine albumin transcript ratios as measured by ddPCR (control n=3, 10wk n=3, 16wk n=5). Panel **(c)** of Figure 5 (Figure 5c) is a graph depicting relative M-AAT-c-Myc serum concentrations as measured by ELISA and normalized to the average of an age-matched control group (control n=6, treated n=10). Panel **(d)** of Figure 5 (Figure 5d) consists of images of mouse liver immunostained using an anti-c-Myc antibody with two representative mice shown from each group (scale bar=2mm). Panel **(e)** of Figure 5 (Figure 5e) is a graph depicting the number of c-Myc positive cells observed in anti-c-Myc immunostained mouse liver (control n=3, 10wk n=3, 16wk n=6). Panel **(f)** of Figure 5 (Figure 5f) is a graph depicting average relative Z-AAT serum concentration measured by ELISA and normalized to the average of an age-matched control group and presented as percentage of silencing (wk8 n=9, wk11-wk16 n=10). The error bars in each graph correspond to standard error.

### DETAILED DESCRIPTION

The singular terms "a," "an," and "the" are not intended to be limiting and include plural referents unless explicitly stated otherwise or the context clearly indicates otherwise.

Various aspects of the present invention relate to the discovery that immunodeficient mice genetically modified to express the PiZ variant of human α-1 antitrypsin provide a favorable environment for the engraftment of human hepatocytes which do not express the PiZ variant of human α-1 antitrypsin. The mice, called genetically modified immunodeficient PiZ mice herein, are useful, for example, as models of human liver function and disease. Genetically modified immunodeficient PiZ mice breed and survive without maintenance drug therapy and can also be used as an *in vivo* model to identify optimal vectors to transduce human hepatocytes, which often differ from mouse hepatocytes in their tropism for specific AAV capsid variants. Genetically modified immunodeficient PiZ mice including human hepatocyte xenografts are also useful as tools for *in vivo* directed evolution studies.

Genetically modified immunodeficient PiZ mice express a PiZ variant of α-1 antitrypsin (AAT) that is the Glu342Lys variant of human α-1 antitrypsin.

The expression of a PiZ variant of α-1 antitrypsin (Z-AAT) in immunodeficient mice was found to provide a surprisingly useful selective advantage for hepatocyte xenografts.

Scientific and technical terms used herein are intended to have the meanings commonly understood by those of ordinary skill in the art. Such terms are defined and used in context in various standard references illustratively including A. Nagy, M. Gertsenstein, K. Vintersten, R. Behringer, Manipulating the Mouse Embryo: A Laboratory Manual, 3rd edition, Cold Spring Harbor Laboratory Press; December 15, 2002, ISBN-10: 0879695919; Kursad Turksen (Ed.), Embryonic stem cells: methods and protocols in Methods Mol Biol. 2002;185, Humana Press; Current Protocols in Stem Cell Biology, ISBN: 9780470151808; Sambrook and Russell, Molecular Cloning: A Laboratory Manual, Cold Spring Harbor Laboratory Press; 3rd Ed., 2001; Ausubel (Ed.), Short Protocols in Molecular Biology, Current Protocols, 5th Ed., 2002; Alberts et al, Molecular Biology of the Cell, 4th Ed., Garland, 2002; Nelson and Cox, Lehninger Principles of Biochemistry, 4th Ed., W.H. Freeman & Company, 2004; and Herdewijn (Ed.), Oligonucleotide Synthesis: Methods and Applications, Methods in Molecular Biology, Humana Press, 2004.

The term "xenogeneic" is used herein with reference to a host cell or organism to indicate that the material referred to as "xenogeneic" is derived from another species than that of the host cell or organism. Xenogeneic material can be, for example, human material, including genes that encode human proteins or cells having substantially human genomes.

The term "allogeneic" is used herein with reference to a host cell or organism to indicate that the material referred to as "allogeneic" is derived from the same species than that of the host cell or organism. Allogeneic material introduced into a host mouse is derived from a mouse, including, for example, genes that encode murine proteins or cells having substantially murine genomes.

The term "comprising" refers to an open group, *e.g.,* a group comprising members A, B, and C can also include additional members. The term "consisting of' refers to a closed group, *e.g.,* a group consisting of members A, B, and C does not include any additional members.

### Genetically Modified Immunodeficient PiZ Mice

The genome of the genetically-modified immunodeficient mouse encodes the PiZ variant of human α-1 antitrypsin (Z-AAT) wherein the PiZ variant of human α-1 antitrypsin has a substitution of lysine for glutamate at amino acid position 342 (Glu342Lys) in the amino acid sequence of the human α-1 antitrypsin protein, SEQ ID NO: 1.

### SEQ ID NO:1

Human Z-AAT protein (without signal peptide) - Glu342Lys

EDPQGDAAQKTDTSHHDQDHPTFNKITPNLAEFAFSLYRQLAHQSNSTNIFFSPVSIAT
AFAMLSLGTKADTHDEILEGLNFNLTEIPEAQIHEGFQELLRTLNQPDSQLQLTTGNGL
FLSEGLKLVDKFLEDVKKLYHSEAFTVNFGDTEEAKKQINDYVEKGTQGKIVDLVKELD
RDTVFALVNYIFFKGKWERPFEVKDTEEEDFHVDQVTTVKVPMMKRLGMFNIQHCKKLS
SWVLLMKYLGNATAIFFLPDEGKLQHLENELTHDIITKFLENEDRRSASLHLPKLSITG
TYDLKSVLGQLGITKVFSNGADLSGVTEEAPLKLSKAVHKAVLTIDKKGTEAAGAMFLE
AIPMSIPPEVKFNKPFVFLMIEQNTKSPLFMGKVVNPTQK

In particular aspects, a nucleotide sequence encoding Z-AAT is integrated into the genome of cell of the mouse. For example, the nucleotide sequence encoding Z-AAT is integrated into the genome of germline cells of the mouse thereby enabling the inheritance of the nucleotide sequence encoding Z-AAT to progeny of the mouse.

A human PiZ variant of human α-1 antitrypsin (Z-AAT) protein is identified herein as SEQ ID NO: 1 and variants thereof are useful in the present invention.

The nucleotide sequence may encode an amino acid sequence that has at least about 85%, 90%, 91%, 92%, 93%, 94%, 95%, 96%, 97%, 98%, 99%, 99.5%, 99.7%, 99.8% or greater, sequence identity with the amino acid sequence set forth in SEQ ID NO: 1. The nucleotide sequence encoding the amino acid sequence may be as set forth in SEQ ID NO: 1.

The genetically modified immunodeficient PiZ mouse may express mRNA encoding Z-AAT having an amino acid sequence that has at least about 85%, 90%, 91%, 92%, 93%, 94%, 95%, 96%, 97%, 98%, 99%, 99.5%, 99.7%, 99.8%, or greater, sequence identity with the amino acid sequence set forth in SEQ ID NO:1. The genetically modified immunodeficient PiZ mouse may express mRNA encoding the amino acid sequence set forth in SEQ ID NO: 1.

The genetically modified immunodeficient PiZ mouse may express a Z-AAT protein that has at least about 85%, 90%, 91%, 92%, 93%, 94%, 95%, 96%, 97%, 98%, 99%, 99.5%, 99.7%, 99.8% or greater sequence identity with the amino acid sequence set forth in SEQ ID NO: 1. The genetically modified immunodeficient PiZ mouse may express a protein with the amino acid sequence set forth in SEQ ID NO: 1.

The nucleic acid encoding Z-AAT may be operably linked to a promoter wherein the Z-AAT includes the amino acid sequence of SEQ ID NO:1, or the amino acid sequence encoded by the complement of a nucleic acid which hybridizes to SEQ ID NO:2 under highly stringent hybridization conditions. It will be appreciated by those of ordinary skill in the art that, due to the degenerate nature of the genetic code, alternate nucleic acid sequences encode Z-AAT and variants thereof and that such alternate nucleic acids may be used in compositions and methods described herein.

### SEQ ID NO:2

Encoding Human Z-AAT protein (without signal peptide) - Glu342Lys

gaggatccccagggagatgctgcccagaagacagatacatcccaccatgatcaggatcacccaaccttcaa
caagatcacccccaacctggctgagttcgccttcagcctataccgccagctggcacaccagtccaacagca
ccaatatcttcttctccccagtgagcatcgctacagcctttgcaatgctctccctggggaccaaggctgac
actcacgatgaaatcctggagggcctgaatttcaacctcacggagattccggaggctcagatccatgaagg
cttccaggaactcctccgtaccctaaaccagccagacagccagctccagctgaccaccggcaatggcctgt
tcctcagcgagggcctgaagctagtggataagtttttggaggatgttaaaaagttgtaccactcagaagcc
ttcactgtcaacttcggggatcacgaagaggccaagaaacagatcaacgattacgtggagaagggtactca
agggaaaattgtggatttggtcaaggagcttgacagagacacagtttttgctctggtgaattacatcttct
ttaaaggcaaatgggagagaccttttgaagtcaaggacaccgaggacgaggacttccacgtggaccaggtg
accaccgtgaaggtccctatgatgaagcgtttaggcatgtttaacatccagcactgtaagaagctgtccag
ctgggtactgctaatgaaatacctgggcaatgccaccgccatcttcttcctacctgatgaggggaaactac
agcacctggaaaatgaactcacccacgatatcatcaccaagttcctggaaaatgaagacagaaggtctgcc
agcttacatttacccaaactgtccattactggaacctatgatctgaagagcgtcctgggtcaactgggcat
cactaaggtcttcagcaatggggctgacctctccggggtcacagaggaggcacccctgaagctctccaagg
ccgtgcataaggctgtgctgaccatcgacaagaaggggactgaagctgctggggccatgtttttagaggcc
ataccaatgtctatccccccagaggtcaagttcaacaaaccctttgtcttcttaatgattgaacaaaatac
caagtctcccctcttcatgggaaaagtggtgaatcccacccaaaaa

To determine the percent identity of two amino acid sequences or two nucleotide sequences, the sequences are aligned for optimal comparison purposes (e.g., gaps can be introduced in a first amino acid or nucleotide sequence for optimal alignment with a second amino acid or nucleotide sequence using the default parameters of an alignment software program). The amino acids or nucleotides at corresponding amino acid positions or nucleotide positions are then compared. When a position in the first sequence is occupied by the same amino acid or nucleotide as the corresponding position in the second sequence, then the sequences are identical at that position. The percent identity between the two sequences is a function of the number of identical positions shared by the sequences (*i.e.,* % identity = number of identical aligned positions ÷ total number of aligned positions · 100%). In some embodiments, the two sequences have the same length.

The determination of percent identity between two sequences can be accomplished using a mathematical algorithm. A non-limiting example of a mathematical algorithm utilized for the comparison of two sequences is the algorithm of Karlin and Altschul, 1990, PNAS USA 87:2264-68, *e.g.*, as modified as in Karlin and Altschul, 1993, PNAS USA 90:5873-77. Such an algorithm is incorporated in the NBLAST and XBLAST programs of Altschul et al, 1990, J. Mol. Biol. 215:403. BLAST nucleotide searches can be performed with the NBLAST nucleotide program parameter set, *e.g.,* for score=100, wordlength=12, to obtain nucleotide sequences homologous to nucleotide sequences of the present invention. BLAST protein searches can be performed with the XBLAST program parameter set, *e.g.,* to score 50, wordlength=3, to obtain amino acid sequences homologous to a protein molecule of the present invention. To obtain gapped alignments for comparison purposes, Gapped BLAST can be utilized as described in Altschul et al, 1997, Nucleic Acids Res. 25:3389-02. Alternatively, PSI BLAST can be used to perform an iterated search that detects distant relationships between molecules (*id*.). When utilizing BLAST, Gapped BLAST, and PSI Blast, the default parameters of the respective programs (*e.g*., of XBLAST and NBLAST) are used (*see, e.g.,* the NCBI website). Another preferred, non-limiting example of a mathematical algorithm utilized to compare sequences is the algorithm of Myers and Miller, 1988, CABIOS 4:11-17. Such an algorithm is incorporated in the ALIGN program (version 2.0) which is part of the GCG sequence alignment software package. When utilizing the ALIGN program for comparing amino acid sequences, a PAM120 weight residue table, a gap length penalty of 12, and a gap penalty of 4 is used. The Clustal suite of software programs provides an additional method for aligning sequences to determine percent sequence identity.

The percent identity between two sequences is determined using techniques similar to those described above with or without allowing gaps. In calculating percent identity, only exact matches are typically counted.

The terms "hybridization" and "hybridizes" refer to pairing and binding of complementary nucleic acids. Hybridization occurs to varying extents between two nucleic acids depending on factors such as the degree of complementarity of the nucleic acids, the melting temperature, Tm, of the nucleic acids and the stringency of hybridization conditions, as is well known in the art. The term "stringency of hybridization conditions" refers to conditions of temperature, ionic strength, and composition of a hybridization medium with respect to particular common additives such as formamide and Denhardt's solution. Determination of particular hybridization conditions relating to a specified nucleic acid is routine and is well known in the art, for instance, as described in J. Sambrook and D.W. Russell, Molecular Cloning: A Laboratory Manual, Cold Spring Harbor Laboratory Press; 3rd Ed., 2001; and F.M. Ausubel, Ed., Short Protocols in Molecular Biology, Current Protocols; 5th Ed., 2002. High stringency hybridization conditions are those which only allow hybridization of substantially complementary nucleic acids. Typically, nucleic acids having about 85-100% complementarity are considered highly complementary and hybridize under high stringency conditions. Intermediate stringency conditions are exemplified by conditions under which nucleic acids having intermediate complementarity, about 50-84% complementarity, as well as those having a high degree of complementarity, hybridize. In contrast, low stringency hybridization conditions are those in which nucleic acids having a low degree of complementarity hybridize.

The terms "specific hybridization" and "specifically hybridizes" refer to hybridization of a particular nucleic acid to a target nucleic acid without substantial hybridization to nucleic acids other than the target nucleic acid in a sample.

Stringency of hybridization and washing conditions depends on several factors, including the Tm of the probe and target and ionic strength of the hybridization and wash conditions, as is well-known to the skilled artisan. Hybridization and conditions to achieve a desired hybridization stringency are described, for example, in Sambrook et al., Molecular Cloning: A Laboratory Manual, Cold Spring Harbor Laboratory Press, 2001; and Ausubel, F. et al., (Eds.), Short Protocols in Molecular Biology, Wiley, 2002.

Mutations can be introduced using standard molecular biology techniques such as site-directed mutagenesis and PCR-mediated mutagenesis. One of skill in the art will recognize that one or more amino acid mutations can be introduced without altering the functional properties of Z-AAT. One or more nucleotide sequences encoding Z-AAT can be altered from the genes and/or mRNA encoding Z-AAT, *e.g.*, to optimize codon usage for a particular mouse or for convenience such as to add or delete restriction sites or to modify sequences that are challenging to clone, amplify, and/or sequence.

Conservative amino acid substitutions can be made in the Z-AAT protein to produce Z-AAT variants. Conservative amino acid substitutions are art recognized substitutions of one amino acid for another amino acid having similar characteristics. For example, each amino acid can be described as having one or more of the following characteristics: electropositive, electronegative, aliphatic, aromatic, polar, hydrophobic, and hydrophilic. A conservative substitution is a substitution of one amino acid having a specified structural or functional characteristic for another amino acid having the same characteristic. Acidic amino acids include aspartate, glutamate; basic amino acids include histidine, lysine, arginine; aliphatic amino acids include isoleucine, leucine, and valine; aromatic amino acids include phenylalanine, tyrosine, and tryptophan; polar amino acids include aspartate, glutamate, histidine, lysine, asparagine, glutamine, arginine, serine, threonine, and tyrosine; and hydrophobic amino acids include alanine, cysteine, phenylalanine, glycine, isoleucine, leucine, methionine, proline, valine, and tryptophan; and conservative substitutions include substitutions among amino acids within each group. Amino acids can also be described in terms of sterics or relative size, *e.g.*, alanine, cysteine, aspartate, glycine, asparagine, proline, threonine, serine, and valine are all typically considered to be small.

The term "nucleic acid" refers to RNA or DNA molecules having more than one nucleotide in any form including single-stranded, double-stranded, oligonucleotide, or polynucleotide. The term "nucleotide sequence" refers to the ordering of nucleotides in a nucleic acid.

Nucleic acids encoding Z-AAT and variants thereof can be isolated or generated recombinantly or synthetically using well-known methodology.

In particular aspects of the present invention, a nucleotide sequence encoding Z-AAT is operably-linked to a promoter. The promoter can be, for example, a constitutive promoter. In particular aspects, the promoter is capable of driving gene expression in a host mouse (*e.g.*, an immunodeficient mouse). For example, the promoter can be a CAG promoter. The CAG promoter includes the cytomegalovirus early enhancer element ("C"), the first exon and the first intron of the chicken beta-actin gene ("A"), and the splice acceptor of the rabbit beta-globulin gene ("G"). The CAG promoter is well known and displays robust expression in mouse cells (*see, e.g.,* Jun-ichi et al, 1989, Gene, 79(2):269). The CAG promoter can be modified, for example, to remove exons. Other promoters are known to drive robust gene expression in mice including the cytomegalovirus (CMV) immediate-early promoter and the simian virus 40 (SV40) early promoter, each of which may be used in various embodiments of the invention. Methods for designing genes and positioning promoters in transgenic constructs are known (*see, e.g.,* Haruyama et al, 2009, Curr. Protoc. Cell Biol., 19.10).

The genetically modified immunodeficient mouse may include in its genome a nucleotide sequence encoding Z-AAT that is operably-linked to a constitutive promoter.

The genetically modified immunodeficient PiZ mouse's genome may include an expression cassette including a nucleotide sequence encoding Z-AAT, wherein the nucleotide sequence is operably-linked to a promoter and a polyadenylation signal, and the mouse expresses the encoded Z-AAT.

The terms "expression construct" and "expression cassette" are used herein to refer to a double-stranded recombinant nucleotide sequence containing a desired coding sequence (*e.g*., a nucleotide sequence encoding Z-AAT) and containing one or more regulatory elements necessary or desirable for the expression of the operably-linked coding sequence. The term "regulatory element" as used herein refers to a nucleotide sequence that controls some aspect of the expression of nucleotide sequences. Exemplary regulatory elements illustratively include an enhancer, an internal ribosome entry site (IRES), an intron, an origin of replication, a polyadenylation signal (pA), a promoter, a transcription termination sequence, and an upstream regulatory domain, which contribute to the replication, transcription, and post-transcriptional processing of a nucleotide sequence. Those of ordinary skill in the art are capable of selecting and using these and other regulatory elements in an expression construct with no more than routine experimentation. Expression constructs can be generated recombinantly or synthetically using well-known methodology.

The term "operably-linked" as used herein refers to a nucleotide sequence in a functional relationship with a second nucleotide sequence.

A regulatory element included in an expression cassette can be a promoter. The term "promoter" as used herein refers to a regulatory nucleotide sequence operably-linked to a coding nucleotide sequence to be transcribed such as a nucleotide sequence encoding a desired amino acid. A promoter is generally positioned upstream of a nucleotide sequence to be transcribed and provides a site for specific-binding by RNA polymerase and other transcription factors. In particular aspects of the present invention, the promoter is a constitutive promoter or an inducible promoter. In particular aspects of the present invention, the promoter provides either ubiquitous, tissue-specific, or cell-type specific expression.

Ubiquitous promoters that can be included in an expression construct include, but are not limited to, a 3-phosphoglycerate kinase (PGK-1) promoter, a beta-actin promoter, a ROSA26 promoter, a heat shock protein 70 (Hsp70) promoter, an EF-1 alpha gene encoding elongation factor 1 alpha (EF-1) promoter, an eukaryotic initiation factor 4A (eIF-4A1) promoter, a chloramphenicol acetyltransferase (CAT) promoter, and a cytomegalovirus (CMV) promoter.

Tissue-specific promoters that can be included in an expression construct include, but are not limited to, a promoter of a gene expressed by hepatocytes.

These and other promoters are known in the art as exemplified in Abboud et al, 2003, J. Histochem & Cytochem., 51(7):941-49; Schorpp et al, 1996, Nucl. Acids Res., 24(9): 1787-88; McBurney et al, 1994, Devel. Dynamics, 200:278-93; and Majumder et al, 1996, Blood, 87(8):3203-11.

In addition to a promoter, one or more enhancer sequences can be included such as, but not limited to, the cytomegalovirus (CMV) early enhancer element and the SV40 enhancer element.

In various aspects of the present invention, additional included sequences include an intron sequence such as the beta globin intron or a generic intron, a transcription termination sequence, and an mRNA polyadenylation (pA) sequence such as, but not limited to SV40-pA, beta-globin-pA, and AAT-pA.

In various aspects of the present invention, an expression construct includes sequences necessary for amplification in bacterial cells, such as a selection marker (e.g., a kanamycin or ampicillin resistance gene) and an origin of replication.

An expression construct can be introduced into fertilized mouse oocytes, a preimplantation mouse embryo or into mouse stem cells (embryonic stem cells or induced pluripotent stem cells) to generate a genetically modified mouse using well-known methods.

For methods of introduction of an expression construct into a mouse preimplantation embryo, the expression construct can be linearized before injection into the embryo.

Preferably, the expression construct is injected into fertilized oocytes. Fertilized oocytes are collected from superovulated females the day after mating (0.5 days post coitum) and injected with the expression construct. The injected oocytes are either cultured overnight or transferred directly into oviducts of 0.5-day post coitum pseudopregnant females. Methods for superovulation, harvesting of oocytes, expression construct injection, and embryo transfer are known in the art and described, for example, in Manipulating the Mouse Embryo: A Laboratory Manual, 3rd edition, Cold Spring Harbor Laboratory Press; December 15, 2002, ISBN-10: 0879695919. Offspring can be tested for the presence of the Z-AAT transgene by DNA analysis, such as by PCR, Southern blot, or nucleotide sequencing. Mice that carry the transgene can be tested for protein expression such as by ELISA or Western blot analysis.

Alternatively, an expression construct is introduced by a technique such as electroporation, calcium-phosphate precipitation, or lipofection. The cells are then screened for transgene integration by DNA analysis, such as PCR, Southern blot, or nucleotide sequencing. Cells with the correct integration can be tested for functional expression by protein analysis for Z-AAT using, for example, ELISA or Western blot analysis.

Embryonic stem cells are grown in media optimized for the particular cell line. Typically, embryonic stem cell media contains 15% fetal bovine serum (FBS) or synthetic or semi-synthetic equivalents, 2 mM glutamine, 1 mM sodium pyruvate, 0.1 mM non-essential amino acids, 50 U/mL penicillin and streptomycin, 0.1 mM 2-mercaptoethanol, and 1000 U/mL LIF (plus, for some cell lines chemical inhibitors of differentiation) in Dulbecco's Modified Eagle Media (DMEM). A detailed description is known in the art (Tremml et al, 2008, Current Protocols in Stem Cell Biology, Chapter 1:Unit 1C.4). For a review of inhibitors of embryonic stem cell differentiation, *see* Buehr et al, 2003, Genesis of embryonic stem cells, Philosophical Transactions of the Royal Society B: Biological Sciences 358:1397-1402.

Selected cells incorporating the expression construct can be injected into preimplantation embryos. For microinjection, embryonic stem cells or induced pluripotent stem cells are rendered to single cells using a mixture of trypsin and EDTA, followed by resuspension in embryonic stem cell media. Groups of single cells are selected using a finely drawn-out glass needle (20-25 micrometer inside diameter) and introduced through the embryo's zona pellucida and into the blastocysts cavity (blastocoel) using an inverted microscope fitted with micromanipulators. Stem cells can also be injected into early stage embryos (*e.g.*, 2-cell, 4-cell, 8-cell, premorula, or morula). Injection may be assisted with a laser or piezo-pulsed drilled opening in the zona pellucida. Approximately 9-10 selected stem cells (embryonic stem cells or induced pluripotent stem cells) are injected per blastocyst, or 8-cell stage embryo, 6-9 stem cells per 4-cell stage embryo, and about 6 stem cells per 2-cell stage embryo. Following stem cell introduction, embryos are allowed to recover for a few hours at 37 °C in 5% CO₂, 5% O₂ in nitrogen or cultured overnight before transfer into pseudopregnant recipient females. In a further alternative to stem cell injection, stem cells can be aggregated with morula stage embryos. All of these methods are well established and can be used to produce stem cell chimeras. For a more detailed description, *see, e.g.,* Manipulating the Mouse Embryo: A Laboratory Manual, 3rd edition (Nagy, Gertsenstein, Vintersten, and Behringer, Cold Spring Harbor Laboratory Press, December 15, 2002, ISBN-10: 0879695919; *see also* Nagy et al, 1990, Development 110:815-821; Kraus et al, 2010, Genesis 48:394-399; and U.S. Patent No's 7,576,259, 7,659,442, and 7,294,754).

Pseudopregnant embryo recipients are prepared using methods known in the art. Briefly, fertile female mice at 6-8 weeks of age are mated with vasectomized or sterile males to induce a hormonal state conductive to supporting surgically introduced embryos. At 2.5 days post coitum (dpc) up to 15 of the stem cell containing blastocysts are introduced into the uterine horn very near to the uterus-oviduct junction. For early stage embryos and morula, such embryos are either cultured *in vitro* into blastocysts or implanted into the oviducts of 0.5 dpc or 1.5 dpc pseudopregnant females according to the embryo stage. Chimeric pups from the implanted embryos are born 16-20 days after the transfer depending on the embryo age at implantation. Chimeric males are selected for breeding. Offspring can be analyzed for transmission of the embryonic stem cell genome, for example, by coat color and/or genetic analysis, such as PCR, Southern blot, or nucleotide sequencing. Protein expression (*e.g.*, of Z-AAT) can be analyzed by protein analysis (Western blot, ELISA) or other functional assays. Offspring expressing the Z-AAT transgene can be intercrossed to create mice homozygous for the transgene. The transgenic mice can be crossed with immunodeficient mice to create a congenic immunodeficient strain expressing a Z-AAT transgene.

A Z-AAT transgene can also be targeted into a specific locus of the stem cell genome that is known to result in reliable expression such as the Hprt or the Rosa26 locus. For targeted transgenics, a targeting construct can be made using recombinant DNA techniques. The targeting construct can optionally include 5' and 3' sequences that are homologous to the endogenous gene target. The targeting construct can optionally further include a selectable marker such as neomycin phosphotransferase, hygromycin, or puromycin, a nucleic acid encoding Z-AAT, and a polyadenylation signal, for example. To ensure correct transcription and translation of a nucleotide sequence encoding a gene, for example, the sequence is either in frame with the endogenous gene locus, or a splice acceptor site and internal ribosome entry site (IRES) sequence are included. Such a targeting construct can be transfected into stem cells, and the stem cells can be screened to detect the homologous recombination event using PCR, Southern blot, or nucleotide sequencing. Cells with the correct homologous recombination event can be further analyzed for transgene expression by protein analysis, such as by ELISA or Western blot analysis. If desired, the selectable marker can be removed, for example, by treating the stem cells with Cre recombinase. After Cre recombinase treatment, the cells are analyzed for the presence of the nucleotide sequence encoding the gene. Cells with the correct genomic event can be selected and injected into preimplantation embryos as described above. Chimeric males are selected for breeding. Offspring can be analyzed for transmission of the embryonic stem cell genome by coat color and genetic analysis, such as PCR, Southern blot, or nucleotide sequencing, and they can be tested for protein expression such as by protein analysis (Western blot, ELISA) or other functional assays. Offspring expressing the protein can be intercrossed to create mice homozygous for the transgene. Transgenic mice can be crossed with immunodeficient mice to create a congenic immunodeficient strain with the transgene.

The transgenic mice may include a Z-AAT transgene in substantially all of their cells, as well as transgenic mice that include a Z-AAT transgene in some, but not all of their cells. In particular aspects of the present invention, one or multiple copies (such as concatamers) of the Z-AAT transgene can be integrated into the genomes of the cells of the transgenic mice.

Any of various methods can be used to introduce a Z-AAT transgene into mice to produce transgenic mice expressing Z-AAT. Such techniques are well-known in the art and include, but are not limited to, pronuclear microinjection and transformation of embryonic stem cells. Methods for generating transgenic mice that can be used include, but are not limited to, those described in Sundberg and Ichiki, (Eds.), Genetically Engineered Mice Handbook, CRC Press, 2006; Hofker and van Deursen, (Eds.), Transgenic Mouse Methods and Protocols, Humana Press, 2002; Joyner, Gene Targeting: A Practical Approach, Oxford University Press, 2000; Manipulating the Mouse Embryo: A Laboratory Manual, 3rd edition, Cold Spring Harbor Laboratory Press, December 15, 2002, ISBN-10: 0879695919; Turksen (Ed.), Embryonic stem cells: methods and protocols in Methods Mol Biol. 2002, 185, Humana Press; Current Protocols in Stem Cell Biology, ISBN: 978047015180; Meyer et al, PNAS USA, 107 (34):15022-26.

Homology-based recombination gene modification strategies can be used to genetically modify an immunodeficient mouse by "knock-in" to introduce a nucleic acid encoding an exogenous protein or proteins e.g., a nucleotide sequence encoding hSCF, a nucleotide sequence encoding hGM-CSF, a nucleotide sequence encoding hIL-3, and a nucleotide sequence encoding hCSF1 into the genome of the immunodeficient mouse, such as homing endonucleases, integrases, meganucleases, transposons, nuclease-mediated processes using a zinc finger nuclease (ZFN), a Transcription Activator-Like (TAL), a Clustered Regularly Interspaced Short Palindromic Repeats (CRISPR)-Cas, or a Drosophila Recombination-Associated Protein (DRAP) approach. See, for example, Cerbini et al., PLoS One. 2015; 10(1): e0116032; Shen et al., PLoS ONE 8(10): e77696; and Wang et al., Protein & Cell, February 2016, Volume 7, Issue 2, pp 152-156.

### Immunodeficiency

The term "immunodeficient mouse" refers to a mouse characterized by one or more of: a lack of functional immune cells, such as T cells and B cells; a DNA repair defect; a defect in the rearrangement of genes encoding antigen-specific receptors on lymphocytes; and a lack of immune functional molecules such as IgM, IgG1, IgG2a, IgG2b, IgG3 and IgA. Immunodeficient mice can be characterized by one or more deficiencies in a gene involved in immune function, such as *Rag1* and *Rag2* (Oettinger, M.A et al., Science, 248:1517-1523, 1990; and Schatz, D. G. et al., Cell, 59:1035-1048, 1989) Immunodeficient mice may have any of these or other defects which result in abnormal immune function in the mice.

According to particular aspects, the genetically modified immunodeficient PiZ mouse has a defect in its endogenous gene encoding interleukin-2 receptor y subunit (IL-2RG) which causes the mouse to express a defective endogenous interleukin-2 receptor y subunit and/or a reduced amount of endogenous interleukin-2 receptor y subunit, or the mouse may not express an endogenous interleukin-2 receptor y subunit at all. The mouse can optionally be IL-2RG null such that it lacks a functional endogenous *IL-2rg* gene.

In further aspects, the genetically modified immunodeficient PiZ mouse has a defect in its endogenous gene encoding DNA-dependent protein kinase, catalytic subunit (Prkdc) which causes the mouse to express a defective endogenous DNA-dependent protein kinase, catalytic subunit and/or a reduced amount of endogenous DNA-dependent protein kinase, catalytic subunit, or the mouse may not express endogenous DNA-dependent protein kinase, catalytic subunit at all. The mouse can optionally be *Prkdc null* such that it lacks a functional endogenous Prkdc gene).

"Endogenous," as used herein in relation to genes and the proteins they encode, refers to genes present in the genome of the mouse at their native gene locus.

In various aspects of the present invention, the genetically modified immunodeficient PiZ mouse is *IL-2rg null* and/or *Prkdc null,* lacking functional endogenous *IL-2Rg* and *Prkdc* genes. The mouse can optionally include a *Prkdc* knockout and/or an *IL-2rg* knockout. In various aspects of the present invention, the mouse does not express either *IL-2rg, Prkdc,* or both *IL-2rg* and *Prkdc.*

In various aspects of the present invention, the genetically modified immunodeficient PiZ mouse has severe-combined immunodeficiency. The term "severe combined immune deficiency" or "SCID" refers to a condition characterized by the absence of T cells and a lack ofB cell function.

Common forms of SCID include: X-linked SCID, which is characterized by gamma chain gene mutations in the *IL-2rg* gene and the lymphocyte phenotype T(-) B(+) NK(-). and autosomal recessive SCID. Autosomal recessive SCID can be characterized by Jak3 gene mutations and the lymphocyte phenotype T(-) B(+) NK(-), ADA gene mutations and the lymphocyte phenotype T(-) B(-) NK(-), IL-7R alpha-chain mutations and the lymphocyte phenotype T(-) B(+) NK(+), CD3 delta or epsilon mutations and the lymphocyte phenotype T(-) B(+) NK(+), RAG1/RAG2 mutations and the lymphocyte phenotype T(-) B(-) NK(+), Artemis gene mutations and the lymphocyte phenotype T(-) B(-) NK(+), and CD45 gene mutations and the lymphocyte phenotype T(-) B(+) NK(+).

According to aspects of the present invention, the genetically modified immunodeficient PiZ mouse has the severe combined immunodeficiency mutation (*Prkdc^{scid}*)*,* commonly referred to as the *scid* mutation. The *scid* mutation is well-known and located on mouse chromosome 16 as described in Bosma et al, 1989, Immunogenetics 29:54-56. Mice homozygous for the *scid* mutation are characterized by an absence of functional T cells and B cells, lymphopenia, hypoglobulinemia, and a normal hematopoetic microenvironment. The *scid* mutation can be detected, for example, by the detection of markers for the *scid* mutation using well-known methods such as PCR or flow cytometry.

The genetically modified immunodeficient mouse according to aspects of the present invention may have an IL-2 receptor gamma chain deficiency. The term "IL-2 receptor gamma chain deficiency" refers to decreased or dysfunctional IL-2 receptor gamma chain. Decreased IL-2 receptor gamma chain can be due to gene deletion or mutation. Decreased IL-2 receptor gamma chain can be detected, for example, by detection of IL-2 receptor gamma chain gene deletion or mutation and/or detection of decreased IL-2 receptor gamma chain expression using well-known methods.

The genetically modified immunodeficient mouse according to aspects of the present invention may have severe combined immunodeficiency or an IL-2 receptor gamma chain deficiency in combination with severe combined immunodeficiency, wherein the genome of the mice includes a nucleotide sequence encoding Z-AAT.

The genetically modified immunodeficient mouse according to aspects of the present invention may have the *scid* mutation or an IL-2 receptor gamma chain deficiency in combination with the *scid* mutation, wherein the genome of the mice includes a nucleotide sequence encoding Z-AAT.

In various aspects of the present invention, the immunodeficient mouse is a genetically modified NSG mouse, a genetically modified NRG mouse or a genetically modified NOG mouse, wherein the genome of the genetically-modified NSG, NRG or NOG mouse includes a nucleotide sequence encoding Z-AAT.

The terms "NOD scid gamma," *"NOD-scid IL2rg^{null}"* and "NSG" are used interchangeably herein to refer to a well-known immunodeficient mouse strain NOD.Cg-*Prkdc^{scid} IL-2rg^{tm1Wjl}*/SzJ, described in detail in Shultz LD et al, 2005, J. Immunol, 174:6477-89. NSG mice combine multiple immune deficits from the NOD/ShiLtJ background, the severe combined immune deficiency *(scid)* mutation, and a complete knockout of the interleukin-2 receptor gamma chain. As a result, NSG mice lack mature mouse T, B, and NK cells, and they are deficient in mouse cytokine signaling pathways. NSG mice are characterized by a lack of mouse IL-2R-y (gamma c) expression, no detectable mouse serum immunoglobulin, no mouse hemolytic complement, no mature mouse T lymphocytes, and no mature mouse natural killer cells.

An NSG mouse that expresses Z-AAT is referred to as a "NSG-PiZ" mouse, which indicates that the genome of the genetically-modified NSG mouse includes a nucleotide sequence encoding Z-AAT on the NOD.Cg-*Prkdc^{scid} IL-2rg^{tm1Wjl}*/SzJ background, and the mouse expresses Z-AAT.

NRG mice are well-known as NOD.Cg-*Rag1^{tm1Mom} Il2rg^{tm1Wjl}*/SzJ, described in detail in Shultz LD et al, 2008 Clin Exp Immunol 154(2):270-84. An NRG mouse that expresses Z-AAT is referred to as a "NRG-PiZ" mouse, which indicates that the genome of the genetically-modified NRG mouse includes a nucleotide sequence encoding Z-AAT on the NOD.Cg-*Rag1^{tm1Mom} Il2rg^{tm1Wjl}*/SzJ background, and the mouse expresses Z-AAT.

NOG mice are well-known as NOD.Cg-*Prkdc^{scid} Il2rg^{tm1Sug}*/JicTac, described in detail in Ito, M. et al., Blood 100, 3175-3182 (2002). A NOG mouse that expresses Z-AAT is referred to as a "NOG-PiZ" mouse, which indicates that the genome of the genetically-modified NOG mouse includes a nucleotide sequence encoding Z-AAT transgenic mouse on the NOD.Cg-*Prkdc^{scid} Il2rg^{tm1Sug}*/JicTac background, and the mouse expresses Z-AAT.

### Hepatocyte Xenografts

According to aspects of the present invention, the genetically modified immunodeficient PiZ mouse of the present invention has a xenograft which includes human hepatocytes.

The hepatocytes can be from any of various sources, such as primary hepatocytes or a transformed hepatocyte cell line.

In various aspects of the present invention, the cells of the xenograft include an expression construct encoding a marker and express the marker. The marker can be, for example, a fluorescent protein such as green fluorescent protein or a fluorescent variant thereof.

The genetically modified immunodeficient PiZ mouse as described herein has human hepatocytes. In particular aspects, at least about 1%, 2%, 3%, 4%, 5%, 6%, 7%, 8%, 9%, 10%, 12%, 15%, 20%, 25%, 30%, 33%, 35%, 40%, 45%, 50%, or more of the hepatocytes of the mouse are xenogeneic hepatocytes, such as human hepatocytes.

The genetically modified immunodeficient PiZ mouse as described herein may contain engrafted xenogeneic hepatocytes that express corresponding xenogenic liver proteins such as the corresponding xenogeneic serum albumin and the corresponding xenogeneic wild-type AAT. The genetically modified immunodeficient PiZ mouse is engrafted with human hepatocytes, and may express detectable levels of human serum albumin and wild-type human AAT because the human hepatocytes express the human serum albumin and wild-type human AAT.

According to aspects of the present invention, the genetically modified immunodeficient PiZ mouse as described herein containing engrafted xenogeneic hepatocytes has, for example, at least about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0 mg/mL, or more human serum albumin. In particular aspects, the genetically modified immunodeficient PiZ mouse as described herein containing engrafted xenogeneic hepatocytes has about 0.01 to about 10 mg/mL, about 0.01 to about 1 mg/mL, about 0.05 to about 5 mg/mL, about 0.01 to about 0.1 mg/mL, about 0.05 to about 0.5 mg/mL, about 0.1 to about 1 mg/mL, or about 0.5 to about 5 mg/mL human serum albumin. The mouse can have, for example, about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 1.1, 1.2, 1.3, 1.4, 1.5 mg/mL, or more human serum albumin.

According to aspects of the present invention, the genetically modified immunodeficient PiZ mouse as described herein containing engrafted xenogeneic hepatocytes has, for example, at least about 100, 200, 300, 400, 500, 572, 600, 700, 800, 900, 1,000, 2,000, 3,000, 4,000, 5,000, 6,000, 7,000, 8,000 mg/mL, or more serum human wild-type AAT. In particular aspects of the present invention, the genetically modified immunodeficient PiZ mouse as described herein containing engrafted xenogeneic hepatocytes has about 100 to about 10,000 mg/mL, about 100 to about 1000 mg/mL, about 500 to about 5,000 mg/mL, about 1000 to about 10,000 mg/mL, about 572 to about 10,000 mg/mL, about 572 to about 9,000 mg/mL, about 572 to about 8,000 mg/mL, about 600 to about 10,000 mg/mL, about 700 to about 10,000 mg/mL, about 2000 to about 10,000 mg/mL, about 2500 to about 10,000 mg/mL, or about 5000 to about 10,000 mg/mL serum human wild-type AAT. The genetically modified immunodeficient PiZ mouse as described herein containing engrafted xenogeneic hepatocytes can have, for example, about 100, 200, 300, 400, 500, 572, 600, 700, 800, 900, 1,000, 2,000, 3,000, 4,000, 5,000, 6,000, 7,000, 8,000, 9,000, 10,000 mg/mL, or more serum human wild-type AAT.

### Methods of making a genetically modified immunodeficient PiZ mouse containing engrafted xenogeneic or engrafted allogeneic hepatocytes

The present invention relates to a method of making a genetically modified immunodeficient PiZ mouse containing engrafted human hepatocytes.

The method includes providing a genetically modified immunodeficient PiZ mouse as described in the claims.

In particular aspects, the method includes providing a genetically modified immunodeficient NSG-PiZ mouse, NRG-PiZ mouse or NOG-PiZ mouse and administering a population of human cells which includes human hepatocytes to the genetically modified immunodeficient NSG-PiZ mouse, NRG-PiZ mouse or NOG-PiZ mouse.

The administered cells optionally express a marker.

In particular aspects, methods of the present invention include administering about 10³ (1000) to about 10⁶ (1,000,000), about 10³ to about 10⁵, about 10⁴ to about 10⁶, about 10⁵ to about 10⁷, about 1 × 10³ to about 1 × 10⁴, about 5 × 10³ to about 5 × 10⁴, about 1 × 10⁴ to about 1 × 10⁵, about 5 × 10⁴ to about 5 × 10⁵, about 1 × 10⁵ to about 1 × 10⁶, about 5 × 10⁵ to about 5 × 10⁶, about 1 × 10⁶ to about 1 × 10⁷, about 2 × 10⁵ to about 5 × 10⁶, or about 5 × 10⁵ to about 2 × 10⁶ human hepatocytes (e.g., primary human hepatocytes) to a genetically modified immunodeficient PiZ mouse. In particular aspects, methods of the present invention include administering at least about 1 × 10², about 2 × 10², about 3 × 10², about 4 × 10², about 5 × 10², about 6 × 10², about 7 × 10², about 8 × 10², about 9 × 10², about 1 × 10³, about 2 × 10³, about 3 × 10³, about 4 × 10³, about 5 × 10³, about 6 × 10³, about 7 × 10³, about 8 × 10³, about 9 × 10³, about 1 × 10⁴, about 2 × 10⁴, about 3 × 10⁴, about 4 × 10⁴, about 5 × 10⁴, about 6 × 10⁴, about 7 × 10⁴, about 8 × 10⁴, about 9 × 10⁴, about 1 × 10⁵, about 2 × 10⁵, about 3 × 10⁵, about 4 × 10⁵, about 5 × 10⁵, about 6 × 10⁵, about 7 × 10⁵, about 8 × 10⁵, about 9 × 10⁵, about 1 × 10⁶, about 2 × 10⁶, about 3 × 10⁶, about 4 × 10⁶, about 5 × 10⁶, about 6 × 10⁶, about 7 × 10⁶, about 8 × 10⁶, about 9 × 10⁶, about 1 × 10⁷, or more human hepatocytes (e.g., primary human hepatocytes) to a genetically modified immunodeficient PiZ mouse. In particular aspects, methods of the present invention include administering at least about 1 × 10², about 2 × 10², about 3 × 10², about 4 × 10², about 5 × 10², about 6 × 10², about 7 × 10², about 8 × 10², about 9 × 10², about 1 × 10³, about 2 × 10³, about 3 × 10³, about 4 × 10³, about 5 × 10³, about 6 × 10³, about 7 × 10³, about 8 × 10³, about 9 × 10³, about 1 × 10⁴, about 2 × 10⁴, about 3 × 10⁴, about 4 × 10⁴, about 5 × 10⁴, about 6 × 10⁴, about 7 × 10⁴, about 8 × 10⁴, about 9 × 10⁴, about 1 × 10⁵, about 2 × 10⁵, about 3 × 10⁵, about 4 × 10⁵, about 5 × 10⁵, about 6 × 10⁵, about 7 × 10⁵, about 8 × 10⁵, about 9 × 10⁵, about 1 × 10⁶, about 2 × 10⁶, about 3 × 10⁶, about 4 × 10⁶, about 5 × 10⁶, about 6 × 10⁶, about 7 × 10⁶, about 8 × 10⁶, about 9 × 10⁶, about 1 × 10⁷, or more human hepatocytes (e.g., primary human hepatocytes) to a genetically modified immunodeficient PiZ mouse.

In particular aspects of inventive methods, administering human hepatocytes to a genetically modified immunodeficient PiZ mouse includes injecting the hepatocytes. Administering the hepatocytes can include, for example, intrasplenic administration such as intrasplenic injection. The route of administration is not particularly limiting so long as administration allows for the exogenously administered human hepatocytes to populate the liver of the mouse.

The genetically modified immunodeficient PiZ mouse is treated to reduce the number of its endogenous hepatocytes. The term "endogenous hepatocytes" refers to hepatocytes present in the genetically modified immunodeficient PiZ mouse which are naturally present in the mouse. The endogenous hepatocytes can be reduced prior to administering the human or allogeneic hepatocytes to the genetically modified immunodeficient PiZ mouse, such as 1, 2, 3, 4, 5, 6, 7, 8, 9, 10, 11, 12, 13, or 14 days prior to administering the human cells to the mouse.

Surprisingly, the engraftment of xenogeneic hepatocytes into a genetically modified immunodeficient PiZ mouse can be facilitated by treatment with a molecular anti-hepatocyte agent, such as a hepatotoxin that is monocrotaline or a pro-apoptotic antibody that is an anti-mouse CD95 antibody. Treatment with a molecular anti-hepatocyte agent was found to be superior to hepatectomy for establishing a subsequent hepatocyte xenograft. Thus, the method of the invention includes treatment of a genetically modified immunodeficient PiZ mouse with a molecular anti-hepatocyte agent, wherein the molecular anti-hepatocyte agent is monocrotaline or anti-mouse CD95 antibody, and does not include performing a surgical hepatectomy (e.g., a partial or full hepatectomy).

Unlike hepatotoxins, which often target both host and xenograft cells, a pro-apoptotic antibody can be species-specific.

In particular aspects of the present invention, reducing the number of endogenous hepatocytes prior to administration of human hepatocytes includes administering the hepatotoxin, monocrotaline, to a genetically modified immunodeficient PiZ mouse. A method of the present invention optionally includes administering about 1 to about 500 mg/kg monocrotaline to a genetically modified immunodeficient PiZ mouse prior to administration of human hepatocytes, such as about 1 to about 10 mg/kg, about 5 to about 50 mg/kg, about 10 to about 100 mg/kg, about 50 to about 500 mg/kg, about 10 to about 200 mg/kg, about 20 to about 100 mg/kg, or about 40 to about 60 mg/kg monocrotaline. A method of the present invention optionally includes administering about 1 mg/kg, 5 mg/kg, 10 mg/kg, 15 mg/kg, 20 mg/kg, 25 mg/kg, 30 mg/kg, 40 mg/kg, 50 mg/kg, 60 mg/kg, 70 mg/kg, 80 mg/kg, 90 mg/kg, or 100 mg/kg monocrotaline to a genetically modified immunodeficient PiZ mouse prior to administration of human hepatocytes.

In particular aspects of the present invention, monocrotaline is administered to the genetically modified immunodeficient PiZ mouse prior to administration of human hepatocytes once or more than once. The hepatotoxin can be administered, for example, about 1, 2, 3, 4, 5, 6, 7, 8, 9, 10, 11, 12, 13, 14, 15, 16, 17, 18, 19, 20, and/or 21 days prior to administration of human hepatocytes to the genetically modified immunodeficient PiZ mouse.

The genetically modified immunodeficient PiZ mouse includes monocrotaline (e.g., in its body or bloodstream). The mouse can have, for example, about 1 to about 500 mg/kg monocrotaline such as about 1 to about 10 mg/kg, about 5 to about 50 mg/kg, about 10 to about 100 mg/kg, about 50 to about 500 mg/kg, about 10 to about 200 mg/kg, about 20 to about 100 mg/kg, or about 40 to about 60 mg/kg monocrotaline (e.g., in its body or bloodstream). The mouse can optionally have about 1 mg/kg, 5 mg/kg, 10 mg/kg, 15 mg/kg, 20 mg/kg, 25 mg/kg, 30 mg/kg, 40 mg/kg, 50 mg/kg, 60 mg/kg, 70 mg/kg, 80 mg/kg, 90 mg/kg, or 100 mg/kg monocrotaline (e.g., in its body or bloodstream).

In particular aspects of the present invention, reducing the number of endogenous hepatocytes includes administering a pro-apoptotic agent that is an anti-CD95 antibody, to a genetically modified immunodeficient PiZ mouse prior to administration of human hepatocytes. A method according to aspects of the present invention includes administering at least about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.5, 3, 4, 5, 6, 7, 8, 9, or 10 µg of an anti-CD95 antibody to a genetically modified immunodeficient PiZ mouse prior to administration of human hepatocytes. A method according to further aspects of the present invention includes administering about 0.1 to about 10 µg, about 0.1 to about 5 µg, about 0.1 to about 2 µg, about 0.5 to about 10 µg, about 0.5 to about 5 µg, about 0.5 to about 2 µg, about 1 to about 10 µg, or about 1 to about 5 µg of an anti-CD95 antibody to a genetically modified immunodeficient PiZ mouse prior to administration of human hepatocytes. A method according to still further aspects of the present invention includes administering about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.5, 3, 4, 5, 6, 7, 8, 9, or 10 µg of an anti-CD95 antibody to a genetically modified immunodeficient PiZ mouse prior to administration of human hepatocytes.

In some aspects of the present invention, the anti-CD95 antibody is administered once or more than once. A pro-apoptotic antibody can be administered, for example, about 1, 2, 3, 4, 5, 6, 7, 8, 9, 10, 11, 12, 13, 14, 15, 16, 17, 18, 19, 20, and/or 21 days prior to administering human cells to a genetically modified immunodeficient PiZ mouse.

In some aspects of the present invention, the genetically modified immunodeficient PiZ mouse includes a pro-apoptotic agent that is an anti-CD95 antibody (e.g., in its body or bloodstream). The pro-apoptotic antibody can increase apoptosis in the Z-AAT-expressing hepatocytes of the mouse, for example, (e.g., thereby providing an additional selective advantage for xenografted hepatocytes). In some aspects of the present invention, the genetically modified immunodeficient PiZ mouse has about 1 µg of an anti-CD95 antibody (e.g., in its body or bloodstream). For example, the mouse has about 1 µg of an anti-mouse CD95 antibody (e.g., in its body or bloodstream). The mouse can optionally have at least about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.5, 3, 4, 5, 6, 7, 8, 9, or 10 µg of an anti-CD95 antibody (e.g., in its body or bloodstream). The mouse can optionally have about 0.1 to about 10 µg, about 0.1 to about 5 µg, about 0.1 to about 2 µg, about 0.5 to about 10 µg, about 0.5 to about 5 µg, about 0.5 to about 2 µg, about 1 to about 10 µg, or about 1 to about 5 µg of an anti-CD95 antibody (e.g., in its body or bloodstream). The mouse can optionally have about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.5, 3, 4, 5, 6, 7, 8, 9, or 10 µg of an anti-CD95 antibody (e.g., in its body or bloodstream).

Embodiments of compositions and inventive methods are illustrated in the following examples. These examples are provided for illustrative purposes.

### EXAMPLES

### Materials and Methods relating to Examples

### Vector Injection for Genome Editing

For the treatment of adult mice, males between 5 and 9 weeks of age received an intravenous injection of 1E12 gc of vector in 200 µL total volume. For the treatment of P1 mice, both males and females were injected intraperitoneally with 2E11 gc of vector. Animals were bled biweekly via sub-mandibular puncture as per our protocol, and serum was stored at -80°C until time of analysis. At endpoint, animals were euthanized by CO2 asphyxiation followed by cervical dislocation as per our protocol. Liver tissue was harvested, consistent sections were fixed for histology, and the remaining tissue was snap-frozen for downstream molecular analysis.

The B6-PiZ mice used for the genome-editing experiments have a high transgene copy number (>10).

### Animal Procedures

All animal procedures were performed according to the guidelines indicated for rodent surgery of the Institutional Animal Care and Use Committee. Aseptic techniques and sterile instruments were used during all procedures. All recipient animals were males, age 6-10 weeks, and they were anesthetized by isoflurane inhalation in a closed ventilation chamber.

### Liver Perfusion/Primary Hepatocyte Isolation

Mice were euthanized and an abdominal incision was made to expose the abdominal cavity, and the viscera were arranged in order to expose the inferior vena cava. A catheter needle was used to puncture the inferior vena cava and inserted ~1 cm into the inferior vena cava. Suture silks were applied to secure the catheter. Pump tubing was attached to the end of the catheter. The portal vein was sliced to allow outflow. The diaphragm was opened, and the inferior vena cava was clamped off above the liver. To perfuse the liver, liver perfusion medium (Life Technologies) was first pumped through the circulatory system for 50 mL, followed by liver digest medium (Life Technologies) for 50 mL. The liver was removed and immersed in ice-cold hepatocyte wash medium (HWM, Life Technologies) allowing for the hepatocytes to diffuse into solution. The cell suspension was drawn up and pipetted through 70 µm mesh and was centrifuged at 50 g for 3min at 4°C. After spinning down the cells, 10 mL of HWM and 10 mL 90% Percoll (GE Healthcare) in phosphate-buffered saline (PBS; Ca2+, Mg2+ free) were added to the solution to remove non-viable cells. The hepatocyte solution was washed three times with 30 mL of HWM.

### ELISA

The mouse sera were collected biweekly pre- and post-engraftment and tested for total AAT, Z-AAT, c-Myc, and human albumin by ELISA. For all assays, high binding 96-well plates (Immulon4, Dyna-tech Laboratories) were coated with the relevant capture antibody in 100 µL Voller's buffer overnight at 4°C. Unless otherwise indicated, all subsequent incubations were done at room temperature for 1 hr, and plates were washed in between steps with PBS 0.05% Tween 20.

### Human AAT ELISA

Blocking was done with 1% milk. Capture antibody is human-specific goat anti-AAT (1:500, Cat#55111, MP Biomedicals); detection antibody is goat anti-hAAT (horseradish peroxidase [HRP]; 1:5,000, Cat#ab191350, Abcam). Standard is AAT, (Cat# 16-16-011609, Athens Research and Technology).

### Z-AAT ELISA

Blocking was done with 1% milk. Capture antibody is a custom antibody; detection antibody is described above.

### c-Myc ELISA

All incubations were done at 37°C. Blocking was done with 5% BSA. Capture antibody is (1:1,000, Cat# ab19234, Abcam).

### Human Albumin ELISA

Human albumin levels were evaluated post-engraftment by using the human Albumin ELISA quantitation set (Bethyl Labs) as described by the manufacturer. For all assays, after incubation with the HRP-con-jugated antibody, 3,3',5,5'-tetramethylbenzidine (TMB) peroxidase substrate (KPL) was applied, and the reactions were stopped by adding 2 N H2SO4 (Fisher Scientific) after 15-30 min. Plates were read at 450 nm on a VersaMax microplate reader (Molecular Devices).

### Immunohistochemistry

Mouse liver was serially sectioned 4 µm.

### Human Albumin Staining

Sections were de-paraffinized and treated by Citra (Biogenex) at 98°C for 30 min. Background Sniper (Biocare Medical) was applied to reduce non-specific background staining. Sections were incubated with rabbit anti-human albumin (1:4,000, Cat#ab2604, Abcam) for 1 hr. Stain was visualized using Mach2 Goat x Rabbit HRP polymer (Biocare Medical), the 3,3'-diaminobenzidine (DAB) chromagen (Biocare Medical) and Churukian-Allison-Tacha (CAT) hematoxylin counterstain (Biocare Medical).

### Human AAT Staining

Slides were de-paraffinized with xylene and re-hydrated through decreasing concentrations of ethanol to water, including an intermediary step to quench endogenous peroxidase activity (3% hydrogen peroxide in methanol). Slides were transferred to 1x Tris-buffered saline (TBS). For enzyme-induced antigen retrieval, sections were heated for 5 min at 37°C, while submerged in Digest-all Trypsin (Invitrogen). Slides were subsequently rinsed in 1x TBS and incubated with a universal protein blocker Sniper (Biocare Medical), for 15 min at room temperature (RT). Slides were rinsed in 1x TBS and incubated for 60 min with a primary rabbit antibody that recognizes human (and not mouse) AAT (1:600, Cat#KDI-A1ATRYPabr, Fitzgerald). Slides were rinsed in 1x TBS, followed by application of conjugated secondary antibody (Mach 2 goat anti-rabbit horse radish peroxidase-conjugated, Biocare Medical) for 30 min at RT. Detection of AAT was achieved by incubating slides in 3'3' diaminobenzidine (Biocare Medical) for 1.5 min at RT. Slides were counterstained with hematoxylin for 30 seconds and mounted with Cytoseal XYL (Richard-Allen Scientific).

### PAS-D Staining

Schiffs reagent was pre-warmed at RT for at least 30 min. Sections were de-paraffinized and rehydrated. Amylase was applied on the slides for 20 min at RT and rinsed with water. Sections were incubated in 0.5% Periodic acid for 10 min and rinsed. Schiffs reagent was applied to the slides for 15 min. Then sections were washed in lukewarm tap water for 5 min. Hematoxylin counterstain was applied for 15 seconds and blue nuclei for 1 min. One slide without amylase treatment acted as the control.

### PSR Staining

PSR staining was performed using the Picrosirius Red Stain Kit (Cat#24901-250, Polysciences Inc.).

### Threshold Analyses

Analyses were carried out blindly by an experienced technician at the University of Florida Pathology Core.

### Model Optimization

Animals were pre-treated with 50 mg/kg MCT (Oakwood Chemical) intraperitoneally 7 days or 14 and 7 days prior to engraftment or treated at the time of engraftment with 1 µg purified NA/LE hamster anti-mouse CD95 antibody (first apoptosis signal [FAS], BD PharMingen) intravenously.

### Flow Cytometry Assay

After dissociation, cells were stained on ice for 30 minutes with anti-mouse/human CD324 (Biolegend) and anti-HLA-ABC (BD Biosciences) antibodies to determine human hepatocyte percentage. Cells were then washed twice with 1x PBS and resuspended in 1x PBS supplemented with 0.5% FBS and 2 mM EDTA. Cells were acquired using FACS-LSRII flow cytometer (BD Biosciences) and analyzed with FlowJo software (version 10.1; Tree Star Inc.).

### ddPCR Assay

Genomic DNA was isolated (Gentra Puregene kit, QIAGEN) from flash-frozen liver and ddPCR (Bio-Rad) was performed according to the manufacturer's recommendations using 50 ng of DNA as input and TaqMan assays detecting GFP and RPP30.

### Statistics

Regression analysis was followed by a two-tailed Pearson R test Two-way ANOVA was performed to determine the effects of time and treatment. Statistical significance of the difference between age-matched control and treated groups was determined with a paired t test. Statistical significance was defined as p < 0.05.

Example 1. Wild-type mouse hepatocytes can populate the liver of a NSG-PiZ mouse.

To determine whether normal hepatocytes could regenerate a PiZ liver, two new mouse strains were created from a PiZ mouse that expresses human Z-AAT. The PiZ mouse was first backcrossed onto the *NOD-scid IL2rg^{null}* (NSG) background. The severely immunocompromised NSG-PiZ strain allows these mice to host human as well as allogeneic mouse hepatocytes without rejection. Hepatocyte donor mice were also created by crossing the transgenic C57BL/^{TG(CAG-EGFP)} mouse strain with either a wild-type C57BL/6 or a C57BL/6-PiZ transgenic mouse. These crosses generated C57BL/^{TG(CAG-EGFP)}-PiZ F1 litters hemizygous for GFP with (PiZ-GFP) or without (wild-type, WT-GFP) the PiZ mutant human α-1 antitrypsin gene, see **Figure 1a****.** As shown in **Figure 1b****,** hepatocytes isolated from the PiZ-GFP mice expressed both the human Z-AAT and GFP genes whereas the WT-GFP mice only expressed GFP. Hepatocytes from these mice were then used to engraft male NSG-PiZ mice by intrasplenic injection of 1,000,000 PiZ-GFP or WT-GFP donor hepatocytes. Eight weeks post-injection, livers from recipient NSG-PiZ mice were examined for donor engraftment by digital droplet PCR (ddPCR) as well as by immunohistology. The quantitative ddPCR data confirms that that the level of donor hepatocyte engraftment in the NSG-PiZ mouse is dependent on whether the donor hepatocytes express the Z-AAT or not. The results in **Figure 1c** show that on average 40% of the hepatocytes of the NSG-PiZ livers were GFP-positive when mice were engrafted with wild-type GFP-expressing normal hepatocytes as compared to only 5% when the donor hepatocytes were expressing GFP and Z-AAT (PiZ-GFP). These results were confirmed by immunohistochemical (IHC) and immunofluorescent staining of host livers where WT-GFP hepatocytes engrafted at a significantly higher proportion as compared to the PiZ-GFP hepatocytes **(****Figure 1d****-g).** GFP immunohistochemical staining and positive pixel count threshold analysis of the images revealed engraftment varied by lobe between 32% and 48% **(Table 1),** but overall, the positive pixel count analysis for histology sections supports the ddPCR results. This data confirms that wild-type hepatocytes have an increased regenerative capacity as compared to Z-AAT-containing hepatocytes and suggests that the Z-AAT-burdened host liver undergoes sufficient turnover to provide wild-type donor hepatocytes with the ability to repopulate it at significant levels.

**Table 1. Wild-type hepatocytes expand faster than hepatocytes expressing Z-AAT (PiZ).**

| **Genotype of host mouse** | **Genotype of donor hepatocytes** | **Treatment** | **Mouse #** | **% GFP (ddPCR)** | | **% GFP (IHC)** | |
|---|---|---|---|---|---|---|---|
| NSG-PiZ | PiZ-GFP | S+PHx | 832 | 10 | 8 | 14 | 11 |
| | | | 848 | 3 | | 12 | |
| | | | 849 | 11 | | 7 | |
| | | S | 833 | 5 | 5 | 16 | 12 |
| | | | 834 | 9 | | 11 | |
| | | | 835 | 3 | | 8 | |
| | WT-GFP | S+PHx | 838 | 42 | 32 | 43 | 45 |
| | | | 840 | 22 | | 46 | |
| | | S | 841 | 35 | 40 | 50 | 48 |
| | | | 842 | 39 | | 47 | |
| | | | 843 | 45 | | 47 | |
| | | | 844 | 38 | | 48 | |
| B6-GFP | None | None | NA | 95 | 98 | NA | NA |
| | | | NA | 100 | | NA | |

| | | | | | | | |
|---|---|---|---|---|---|---|---|
| B6: C57BL/6, GFP: green fluorescent protein, WT: wild-type, S: splenic injection only, S+PHx: splenic injection and partial hepatectomy, ddPCR: droplet digital polymerase chain reaction, IHC: immunohistochemistry, NA: not available. | | | | | | | |

Example 2. Human hepatocytes can populate the liver of a NSG-PiZ mouse.

NSG-PiZ and NSG control mice were injected intrasplenically with 1,000,000 mature human hepatocytes. Human albumin levels were monitored prospectively in mouse sera samples as a surrogate for human liver engraftment. Serum human albumin levels in **Figure 2a** confirm that liver chimerism occurs as a result of engraftment of human hepatocytes in these mice and that the level of human hepatocyte engraftment achieved in NSG mouse livers expressing Z-AAT increased dramatically. This further confirms the role of Z-AAT burden in creating a niche whereby wild-type hepatocytes are able to expand preferentially.

Given that the human hepatocytes also benefited from an increased mitotic index in the NSG-PiZ mice, increasing hepatocyte turnover may lead to increased engraftment. To test this hypothesis, the experiments were repeated with an added partial hepatectomy at the time of the intrasplenic injections. Because original liver mass generally regenerates in about 7 days after a 70% partial hepatectomy, this proliferative phase was hypothesized to display increased engraftment. As shown in **Figure 2b** partial hepatectomy resulted in a 5-10 fold increase in circulating serum human albumin levels. Interestingly, in the NSG mice hepatectomy caused a decrease in engraftment. This observation may be caused by the advantage of wild-type proliferating mouse hepatocytes over human hepatocytes in the context of a mouse liver. Immunohistochemical staining for human albumin in the NSG-PiZ mouse livers **(****Figure 2c****-d)** confirmed that human hepatocytes are able to engraft a significant portion of the NSG-PiZ livers.

Example 3. Z-AAT burden in NSG-PiZ mice decreases with age, and hepatocyte engraftment correlates with Z-AAT levels.

PiZ transgenic mice have Z-AAT globule-containing hepatocytes that have a higher incidence of apoptosis, and these cells tend to be outcompeted by hepatocytes with lower Z-AAT globule loads. This phenomenon may be the underlying basis for the competitively-advantaged engraftment of wild-type mouse or human hepatocytes in NSG-PiZ mice (although no putative or confirmed mechanism identified herein limits the scope of the patent claims or the disclosure). To more clearly define the consequences of this normal hepatocyte turnover in the absence of donor hepatocytes, turnover was investigated in NSG-PiZ mice, which would also presumably also correlate with decreased Z-AAT globule load over time. A prospective analysis of serum Z-AAT levels in these mice shows a clear and strong inverse correlation (r²=0.80, p<0.003, 2-tailed) between the age of the mice and the Z-AAT serum levels **(****Figure 3a****).** As NSG-PiZ mice age, serum Z-AAT concentrations decrease, which is also reflected histologically by the decrease in the globule-containing status of the hepatocytes between 6 and 14 weeks of age. Globule histology can be visualized, for example, by diastase resistant periodic acid-Schiff (PASD, **Figure 3b****-c)** and human AAT staining of livers at different ages **(****Figure 3d****-e).** This data suggests that, in the absence of engrafted wild-type hepatocytes, the liver in NSG-PiZ mice still exert enough selective pressure to favor the survival and expansion of hepatocytes that produce and accumulate less Z-AAT. Interestingly, as the mice age, some hepatocytes continue to accumulate Z-AAT globules, and it appears that, even though they are scarcer, their globules tend to be larger **(****Figure 3b****).** As the Z-AAT burden decreases with mouse age, the proliferative advantage of the normal human engrafted hepatocytes may also decrease. To investigate human hepatocyte persistence, a retrospective analysis compared the average serum human albumin concentration of a given mouse to the serum Z-AAT concentration at the time of engraftment. This analysis revealed a moderate yet highly significant positive correlation (r²=0.46, p≤0.0003) between Z-AAT serum concentration and the level of human liver chimerism as determined by average human albumin serum concentration **(****Figure 3f****).**

Example 4. Reducing endogenous mouse hepatocytes with small molecules and biologics.

To investigate additional methods for increased human hepatocyte engraftment, two approaches used in the liver xenograft field were tested: monocrotaline (MCT) and a mouse-specific anti-Fas antigen (CD95) antibody. MCT is a pyrrolizidine plant alkaloid with known endothelial toxicity in the lung, liver, and kidney. MCT treatment has been shown to increase cell engraftment in the liver. The anti-CD95 antibody recognizes mouse Fas and leads to cytolytic activity of cells expressing mouse Fas by inducing apoptosis of mouse hepatocytes and other cells. Various protocols employing these two liver injury models were tested and resulted in a "two hit" protocol that can be easily and reproducibly performed without the need for a partial hepatectomy.

Engraftment was significantly increased with both of these interventions and achieved levels comparable or higher than partial hepatectomy **(****Figure 4a****).** Either one or two doses of MCT (50 mg/kg) were administered prior to engraftment, and the greatest engraftment was achieved when mice received two MCT doses **(****Figure 4a****).** The kinetics of engraftment following a single intraperitoneal injection of the anti-CD95 antibody at the time of hepatocyte delivery was just as efficient as the partial hepatectomy as determined by serum human albumin concentration **(****Figure 4a****).** Staining of the MCT- and CD95-treated mouse livers for human albumin confirmed the engraftment of the human hepatocytes **(****Figure 4b****).** Furthermore, a quantitative flow cytometry analysis using an anti-HLA antibody suggests that at least 25% of all hepatocytes in the MCT-treated mice are of human origin **(****Figure 4c****).**

The FDA-accepted therapeutic serum threshold for protein augmentation of human AAT is currently set at 11 µM, equivalent to 572 µg/ml. The results from the xenograft experiments indicate for the first time that this threshold can be achieved with a simple hepatocyte cell therapy approach. Quantification of total human AAT levels after human hepatocyte transplantation in the NSG-PiZ mice shows that it is possible to achieve this therapeutic threshold, and this strategy may be clinically-relevant when combined with a partial hepatectomy **(****Figure 4d****).**

Example 5. Selective expansion of genome-edited hepatocytes.

Male B6-PiZ mice were injected intravenously with either saline or AAV-GeneRide-DualFunctionAAT (GR-dfAAT). GR-dfAAT is a promoterless cassette containing a 2A-peptide sequence followed by a de-targeted, c-Myc-tagged human AAT sequence, and an artificial miRNA targeting Z-AAT (Mueller et al, 2012, Mol. Ther. 20:590-600). The cassette is flanked by two homology arms (1.1-1.3kb) that are complementary to the C57BL/6 *Alb* locus and allow homologous recombination. Following homologous recombination, *Alb* and *AAT* are co-transcribed as a single *Alb-AAT* mRNA ("fused transcript") and produce two proteins through ribosomal skipping **(****Figure 5a****).**

To measure the expression imparted by the genome-edited alleles, the relative abundance of the fused mRNA transcript was quantified by ddPCR as compared to the normal albumin transcript in a subset of mice at week 10 and week 16 post-injection **(****Figure 5b****).** At week 10, the ratio was 0.2%, which increased to 3% at week 16 with one mouse as high as 12.8% **(****Figure 5b****).** This result suggests an expansion of the genome-edited hepatocytes as a function of time. Serum c-Myc-tagged AAT (M-AAT-c-Myc) concentration measurements corroborated these results. Mice were bled prior to injection and biweekly thereafter, which allowed for monitoring of M-AAT-c-Myc serum concentrations by ELISA. The concentrations of M-AAT-c-Myc were slightly increased at week 1 and increased on average 10-fold over time between week 1 and week 16 **(****Figure 5c****).** This increase is statistically significant both across time (p<0.0001) and between control and treated mice (p≤0.0002).

Results were corroborated by immunohistochemistry in a subset of livers stained for c-Myc at 10 and 16 weeks post-injection **(****Figure 5d****).** The staining showed clusters of hepatocytes throughout the entire liver **(****Figure 5d****),** and the size of the clusters increased between the two timepoints. This qualitative observation is supported by a quantitative threshold analysis **(****Figure 5e****),** which calculated the percentage of c-Myc positive pixel counts at 1.6% at week 10 and 2.6% at week 16. Finally, levels of serum Z-AAT were evaluated by ELISA, and the percentage of Z-AAT silencing increased over time **(****Figure 5f****),** which was statistically significant at week 16 (p≤0.0184). Interestingly, the magnitude of Z-AAT silencing was unexpected given that it far exceeds the amount of genome-edited hepatocytes. This finding has important implications and suggests that not all hepatocytes produce equal amounts of Z-AAT. The data may suggest that the highest degree of proliferative advantage for the GeneRide (and thus Z-AAT silencing) may be in those hepatocytes that continue to produce and retain high amounts of Z-AAT. Alternatively, the data may suggest inverted terminal repeat(ITR)-driven expression of the anti-AAT miRNA in unedited hepatocytes. Taken altogether, the data supports the hypothesis that homologous recombination occurred in cells throughout the liver, that the genome-edited hepatocytes then expanded, and that their expansion results in an increase of serum M-AAT with a concomitant decrease of Z-AAT over time.

Further, the data support use of NSG-PiZ mice as a pre-clinical model for gene therapy using genome edited hepatocytes.

Example 6. Development of the NSG-PiZ mouse.

NSG-PiZ mice were prepared by and obtained from the research colonies of Leonard D. Shultz at The Jackson Laboratory. FVB.Cg-Tg(SERPINA1E342K^{∗})#Slcw mice carrying the PiZ allele of the serpin peptidase inhibitor, clade A (alpha-1 antiproteinase, antitrypsin), member 1 [*Homo sapiens*] transgene were obtained from David Perlmutter (University of Pittsburgh School of Medicine). This transgenic model possesses the Z variant (PiZ) allele of the human alpha-1 antitrypsin (SERPINA1*) transgene allowing for human Z-AAT expression. The PiZ allele was back-crossed 5 generations by a marker-assisted speed congenic approach to the NSG strain background. NSG-Tg(SERPINA1^{∗}E342K)#Slcw/Sz, is abbreviated as NSG-PiZ mice. The PiZ transgenic hemizygous mice were intercrossed to fix the PiZ allele to homozygosity. NSG-PiZ homozygotes were raised in a SPF barrier mouse room at The Jackson Laboratory and maintained by intercrossing homozygous breeders. The mice used for engraftment experiments were maintained on sulfamethoxazole/trimethoprim in drinking water on alternate weeks as described in Shultz et al, 2005, J Immunol 174:6477-89. Mice were housed with 12 hr light/ dark cycles on Bed-o'cob 1/4" bedding (Andersons), and fed a standard laboratory diet. Mice were not fasted before any of the procedures. All procedures were carried out during the light cycle.

Example 7. Surgical procedures: intrasplenic injection and partial hepatectomy.

All recipient mice were males, age 6-10 weeks, and they were anesthetized by isoflurane inhalation in a closed ventilation chamber. For intrasplenic injection, an incision was made on mouse left paralumbar fossa in order to expose the spleen. One million primary human hepatocytes (Bioreclamation IVT) suspended in 50 µl of Hanks Balanced Saline solution were injected into the inferior pole of the spleen, using a 25-gauge needle connected to a 1/3 mL syringe. The exposed spleen was returned to the abdominal cavity and the incision was closed with an absorbable suture (4-0 Vicryl). Vet Bond was applied topically to seal the skin incision. For partial hepatectomy, a median-line incision was made to expose the liver. The median lobe of the liver and the left lateral lobe were securely tied off with 4-0 silk Blake braided suture and excised. Two-thirds of the total liver was removed by partial hepatectomy. The remaining liver was gently returned to the abdominal cavity. Finally, the opposing sections of the abdominal wall were aligned at the ventral midline, and an absorbable suture (4-0 Vicryl) was applied to close the incision. The skin incision was sealed by topical application of Vet Bond.

Human hepatocytes administered to mice expressing Z-AAT resulted in mice with livers wherein about 35-50% of the liver cells were human hepatocytes. These mice expressed >0.1 mg/mL serum human albumin and >572 µg/mL serum human AAT. 572 µg/mL (*i.e.,* 11 µM) is notably the FDA-accepted therapeutic serum threshold for protein augmentation of human AAT.

### Example 8

### Genome Editing Improves Liver Phenotype

A promoterless adeno-associated virus (AAV) vector, expressing a wild-type AAT and a synthetic miRNA to silence the endogenous allele, was integrated into the albumin locus. This gene-editing approach leads to a selective advantage of edited hepatocytes, by silencing the mutant protein and augmenting normal AAT production, and improvement of the liver pathology.

The Z-AAT liver pathology is caused by polymerization of the Z-AAT protein and accumulation of those polymers in globules. Clinically the patients present with liver fibrosis, which may lead to cirrhosis and hepatocellular carcinoma. Levels of serum Z-AAT were evaluated by ELISA, and the percentage of Z-AAT silencing increases over time and is statistically significant starting at 6 months in adult-treated mice (two-tailed unpaired t test, p < 0.0002) and starting at 4 months in P1-treated mice (two-tailed unpaired t test, p < 0.0001). Consistent with the M-AAT-c-Myc data, silencing is higher in the P1-treated animals than in the adult-treated animals (two-way ANOVA, p < 0.0012). Interestingly, the magnitude of Z-AAT silencing was unexpected given that it far exceeds the amount of genome-edited hepatocytes. This finding has important implications and suggests that not all hepatocytes produce equal amounts of Z-AAT, and perhaps, as suggested by our data, the highest degree of proliferative advantage for the GeneRide (and thus Z-AAT silencing) may be in those hepatocytes that continue to produce and retain high amounts of Z-AAT. Alternatively, this could point to the inverted terminal repeat (ITR)-driven expression of the anti-AAT miRNA in unedited hepatocytes.

The liver phenotype was further characterized in P1-treated mice, at 6 months and 8 months post-treatment. A periodic acid-Schiff-diastase (PAS-D) staining was performed on liver sections from those mice, in order to assess the amount of Z-AAT globules that appear as magenta spheres. As quantified, the percentage of PAS-D+ cells is significantly reduced by more than 100-fold at both 6 and 8 months post-treatment (two-way ANOVA, p < 0.0001).

A Picro Sirius Red (PSR) immunohistochemistry staining was performed on liver sections from the male animals, in order to assess the amount of collagen fibers, which will appear in red, as a measure of liver fibrosis. Quantification of PSR+ cells showed a reduction of over 40% at 8 months post-treatment (two-tailed unpaired t test, non-significant). Representative images for the PAS-D staining and for the PSR staining were obtained.

Taken all together, the data supports the hypothesis that HR occurred in cells throughout the liver, that the genome-edited hepatocytes then selectively expanded, and that this expansion results in an increase of serum M-AAT with a concomitant decrease of Z-AAT over time. This results in a marked improvement of the liver pathology.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

## Claims

1. A method of making a genetically-modified immunodeficient mouse comprising exogenous hepatocytes, the method comprising:
administering to an immunodeficient genetically-modified mouse a molecular anti-hepatocyte agent, wherein the molecular anti-hepatocyte agent is monocrotaline or anti-mouse CD95 antibody,
wherein the immunodeficient mouse expresses a PiZ variant Glu342Lys of human α-1 antitrypsin (Z-AAT); and
administering to the immunodeficient mouse human hepatocytes;
wherein the molecular anti-hepatocyte agent is administered to the immunodeficient mouse prior to administration of the human hepatocytes; and
wherein the method does not include performing a surgical hepatectomy.

2. A method of assessing the safety and/or efficacy of a putative treatment of a human liver disorder, the method comprising:
administering to an immunodeficient genetically-modified mouse a molecular anti-hepatocyte agent, wherein the molecular anti-hepatocyte agent is monocrotaline or anti-mouse CD95 antibody,
wherein the immunodeficient mouse expresses a PiZ variant Glu342Lys of human α-1 antitrypsin (Z-AAT);
administering to the immunodeficient mouse human hepatocytes,
wherein the molecular anti-hepatocyte agent is administered to the immunodeficient mouse prior to administration of the human hepatocytes, and
wherein the method does not include performing a surgical hepatectomy;
administering to the immunodeficient mouse a putative treatment of a human liver disorder; and
assessing an effect of the putative treatment on the human hepatocytes of the mouse.

3. The method of claim 1, wherein 10-100 mg/kg of the monocrotaline is administered.

4. The method of claim 1, wherein at least 1 × 10⁶ human hepatocytes are administered to the immunodeficient mouse.

5. The method of claim 1, wherein the human hepatocytes comprise genome-edited human hepatocytes.

6. The method of claim 1, wherein the human hepatocytes express wild-type human α-1 antitrypsin (AAT) and human serum albumin.

7. The method of claim 6, wherein the level:
(i) of human serum albumin in serum of the immunodeficient mouse is at least 10 µg/mL; and/or
(ii) of human α-1 antitrypsin in serum of the immunodeficient mouse is at least 100 µg/mL.

8. The method of claim 7, wherein at least 25% of hepatocytes of the mouse are engrafted human hepatocytes that express wild-type human α-1 antitrypsin and human serum albumin.

9. The method of claim 1, wherein the human hepatocytes express a marker protein, optionally wherein the marker protein is green fluorescent protein or a fluorescent analog thereof.

10. The method of claim 2, wherein the putative treatment comprises:
(i) genome-edited human hepatocytes; or
(ii) recombinant viral gene therapy vector.

## Patentansprüche

1. Das Verfahren zur Gewinnung einer genetisch modifizierten immundefizienten Maus, die exogene Hepatozyten aufweist, wobei das Verfahren umfasst:
die Verabreichung eines molekularen Mittels gegen Hepatozyten an eine immundefiziente genetisch modifizierte Maus, wobei das molekulare Mittel gegen Hepatozyten Monocrotalin oder ein anti-Maus CD95 Antikörper ist,
wobei die immundefiziente Maus eine PiZ-Variante Glu342Lys des menschlichen α-1-Antitrypsins (Z-AAT) exprimiert; und
die Verabreichung von menschlichen Hepatozyten an die immundefiziente Maus;
wobei das molekulare Mittel gegen Hepatozyten der immundefizienten Maus vor der Verabreichung der menschlichen Hepatozyten verabreicht wird; und wobei das Verfahren nicht die Durchführung einer chirurgischen Hepatektomie umfasst.

2. Das Verfahren zur Bewertung der Sicherheit und/oder Wirksamkeit einer vermeintlichen Behandlung einer menschlichen Lebererkrankung, wobei das Verfahren umfasst:
die Verabreichung eines molekularen Mittels gegen Hepatozyten an eine immundefiziente genetisch modifizierte Maus, wobei das molekulare Mittel gegen Hepatozyten Monocrotalin oder ein anti-Maus CD95 Antikörper ist,
wobei die immundefiziente Maus eine PiZ-Variante Glu342Lys des menschlichen α-1-Antitrypsins (Z-AAT) exprimiert;
die Verabreichung von menschlichen Hepatozyten an die immundefiziente Maus,
wobei das molekulare Mittel gegen Hepatozyten der immundefizienten Maus vor der Verabreichung der menschlichen Hepatozyten verabreicht wird, und wobei das Verfahren nicht die Durchführung einer chirurgischen Hepatektomie umfasst;
die Verabreichung einer vermeintlichen Behandlung für eine menschliche Lebererkrankung an die immundefiziente Maus; und
die Bewertung einer Wirkung der vermeintlichen Behandlung auf die menschlichen Hepatozyten der Maus.

3. Das Verfahren nach Anspruch 1, wobei 10-100 mg/kg Monocrotalin verabreicht werden.

4. Das Verfahren nach Anspruch 1, wobei mindestens 1 × 10⁶ menschliche Hepatozyten an die immundefiziente Maus verabreicht werden.

5. Das Verfahren nach Anspruch 1, wobei die menschlichen Hepatozyten genom-editierte menschliche Hepatozyten umfassen.

6. Das Verfahren nach Anspruch 1, wobei die menschlichen Hepatozyten menschliches α-1-Antitrypsin (AAT) vom Wildtyp und menschliches Serumalbumin exprimieren.

7. Das Verfahren nach Anspruch 6, wobei der Gehalt:
(i) an Humanserumalbumin im Serum der immundefizienten Maus mindestens 10 µg/ml beträgt; und/oder
(ii) an menschlichem α-1-Antitrypsin im Serum der immundefizienten Maus mindestens 100 µg/ml beträgt.

8. Das Verfahren nach Anspruch 7, wobei mindestens 25% der Hepatozyten der Maus transplantierte menschliche Hepatozyten sind, die menschliches α-1-Antitrypsin vom Wildtyp und menschliches Serumalbumin exprimieren.

9. Das Verfahren nach Anspruch 1, wobei die menschlichen Hepatozyten ein Markerprotein exprimieren, wobei es sich bei dem Markerprotein optional um ein grün fluoreszierendes Protein oder ein fluoreszierendes Analogon davon handelt.

10. Das Verfahren nach Anspruch 2, wobei die vermeintliche Behandlung umfasst:
(i) genom-editierte menschliche Hepatozyten; oder
(ii) rekombinanten viralen Gentherapievektor.

## Revendications

1. Procédé de fabrication d'une souris immunodéficiente génétiquement modifiée comprenant des hépatocytes exogènes, le procédé comprenant :
l'administration à une souris génétiquement modifiée immunodéficiente d'un agent anti-hépatocyte moléculaire, dans lequel l'agent anti-hépatocyte moléculaire est la monocrotaline ou un anticorps CD95 anti-souris,
dans lequel la souris immunodéficiente exprime un variant PiZ Glu342Lys de l'antitrypsine a-1 humaine (Z-AAT) ; et
l'administration à la souris immunodéficiente d'hépatocytes humains ;
dans lequel l'agent anti-hépatocyte moléculaire est administré à la souris immunodéficiente avant l'administration des hépatocytes humains ; et
dans lequel le procédé ne comporte pas la réalisation d'une hépatectomie chirurgicale.

2. Procédé d'évaluation de l'innocuité et/ou de l'efficacité d'un traitement putatif d'un trouble hépatique humain, le procédé comprenant :
l'administration à une souris génétiquement modifiée immunodéficiente d'un agent anti-hépatocyte moléculaire, dans lequel l'agent anti-hépatocyte moléculaire est la monocrotaline ou un anticorps CD95 anti-souris,
dans lequel la souris immunodéficiente exprime un variant PiZ Glu342Lys de l'antitrypsine a-1 humaine (Z-AAT) ;
l'administration à la souris immunodéficiente d'hépatocytes humains,
dans lequel l'agent anti-hépatocyte moléculaire est administré à la souris immunodéficiente avant l'administration des hépatocytes humains, et
dans lequel le procédé ne comporte pas la réalisation d'une hépatectomie chirurgicale ;
l'administration à la souris immunodéficiente d'un traitement putatif d'un trouble hépatique humain ; et
l'évaluation d'un effet du traitement putatif sur les hépatocytes humains de la souris.

3. Procédé selon la revendication 1, dans lequel 10 à 100 mg/kg de la monocrotaline sont administrés.

4. Procédé selon la revendication 1, dans lequel au moins 1 × 10⁶ hépatocytes humains sont administrés à la souris immunodéficiente.

5. Procédé selon la revendication 1, dans lequel les hépatocytes humains comprennent des hépatocytes humains à génome modifié.

6. Procédé selon la revendication 1, dans lequel les hépatocytes humains expriment l'antitrypsine a-1 humaine (AAT) de type sauvage et la sérumalbumine humaine.

7. Procédé selon la revendication 6, dans lequel le niveau :
(i) de sérumalbumine humaine dans le sérum de la souris immunodéficiente est d'au moins 10 µg/ml ; et/ou
(ii) d'antitrypsine a-1 humaine dans le sérum de la souris immunodéficiente est d'au moins 100 µg/ml.

8. Procédé selon la revendication 7, dans lequel au moins 25 % d'hépatocytes de la souris sont des hépatocytes humains greffés qui expriment l'antitrypsine a-1 humaine de type sauvage et la sérumalbumine humaine.

9. Procédé selon la revendication 1, dans lequel les hépatocytes humains expriment une protéine marqueur, éventuellement dans lequel la protéine marqueur est une protéine fluorescente verte ou un analogue fluorescent de celle-ci.

10. Procédé selon la revendication 2, dans lequel le traitement putatif comprend :
(i) des hépatocytes humains à génome modifié ; ou
(ii) un vecteur de thérapie génique virale recombinante.
